(19) Europäisches Patentamt
European Patent Office
Office européen des brevets

(11) Veröffentlichungsnummer: **0 236 263 B1**

(12) **EUROPÄISCHE PATENTSCHRIFT**

(45) Veröffentlichungstag der Patentschrift: **30.12.92**

(51) Int. Cl.[5]: **C07D 403/06**, C07D 207/26, C07D 207/27, A61K 31/40, A61K 31/495

(21) Anmeldenummer: **87810045.2**

(22) Anmeldetag: **26.01.87**

Die Akte enthält technische Angaben, die nach dem Eingang der Anmeldung eingereicht wurden und die nicht in dieser Patentschrift enthalten sind.

# (54) Substituierte Pyrrolidinone.

(30) Priorität: **30.01.86 CH 346/86**

(43) Veröffentlichungstag der Anmeldung: **09.09.87 Patentblatt 87/37**

(45) Bekanntmachung des Hinweises auf die Patenterteilung: **30.12.92 Patentblatt 92/53**

(84) Benannte Vertragsstaaten: **AT BE CH DE ES FR GB GR IT LI LU NL SE**

(56) Entgegenhaltungen:
**EP-A- 0 089 900**
**FR-A- 2 382 441**

(73) Patentinhaber: **CIBA-GEIGY AG**
**Klybeckstrasse 141**
**CH-4002 Basel(CH)**

(72) Erfinder: **Bencze, William, Dr.**
**Scheltenstrasse 6**
**CH-4106 Therwil(CH)**
Erfinder: **Fröstl, Wolfgang, Dr.**
**St. Johanns-Vorstadt 6/2**
**CH-4056 Basel(CH)**
Erfinder: **Wilhelm, Max, Dr.**
**Mohrhaldenstrasse 166A**
**CH-4125 Riehen(CH)**

**EP 0 236 263 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte Pyrrolidin-2-one der Formel

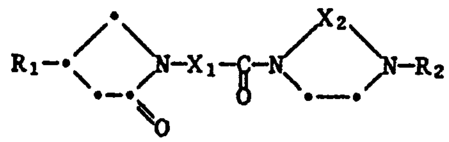

(I),

worin $R_1$ einen unsubstituierten oder durch Niederalkyl, Niederalkoxy, Halogen und/oder Trifluormethyl substituierten Phenyl- oder Naphthylrest bedeutet, $X_1$ für Niederalkyliden steht, $X_2$ Methylen, Aethylen oder Oxoäthylen bedeutet und $R_2$ Wasserstoff, Niederalkyl oder einen Rest der Formel

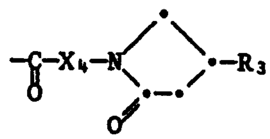

(Ia),

darstellt, in dem $X_4$ Niederalkyliden und $R_3$ Wasserstoff oder einen unsubstituierten oder durch Niederalkyl, Niederalkoxy, Halogen und/oder Trifluormethyl substituierten Phenyl- oder Naphthylrest bedeutet, in freier Form oder in, insbesondere pharmazeutisch verwendbarer, Salzform, Verfahren zur Herstellung der erfindungsgemässen Verbindungen, diese enthaltende pharmazeutische Präparate und ihre Verwendung als Arzneimittelwirkstoffe.

Die Reste $X_1$ und $X_4$ können dabei gleich oder verschieden sein. Ebenso kann ein gegebenenfalls substituierter Phenyl- oder Naphthylrest $R_3$ mit $R_1$ identisch oder von $R_1$ verschieden sein.

Der Phenyl- bzw. Naphthylrest $R_1$ und gegebenenfalls $R_3$ kann unsubstituiert oder wie angegeben einfach oder durch gleiche oder verschiedene der angegebenen Substituenten zwei- oder mehrfach, z.B. mono-, di- oder trisubstituiert, sein und steht beispielsweise für gegebenenfalls wie angegeben substituiertes, insbesondere mono- oder disubstituiertes, Phenyl oder unsubstituiertes Naphthyl, z.B. 1-oder in zweiter Linie 2-Naphthyl.

Der Rest $X_2$ bedeutet insbesondere Aethylen, kann aber, wie erwähnt, auch Oxoäthylen oder Methylen sein.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise $C_1$-$C_7$-Alkyl, vorzugsweise $C_1$-$C_4$-Alkyl, wie Methyl, Aethyl, Propyl, Isopropyl oder Butyl, kann aber auch Isobutyl, Sekundärbutyl, Tertiärbutyl oder eine Pentyl-, Hexyl-oder Heptylgruppe sein.

Niederalkoxy ist beispielsweise $C_1$-$C_7$-Alkoxy, vorzugsweise $C_1$-$C_4$-Alkoxy, wie Methoxy, Aethoxy, Propyloxy, Isopropyloxy oder Butyloxy, kann aber auch Isobutyloxy, Sekundärbutyloxy, Tertiärbutyloxy oder eine Pentyloxy-, Hexyloxy- oder Heptyloxygruppe sein.

Halogen ist beispielsweise Halogen der Atomnummer bis und mit 35, wie Chlor oder Fluor, ferner Brom.

Niederalkyliden ist beispielsweise terminal gebundenes Niederalkyliden, wie entsprechendes $C_1$-$C_7$-Alkyliden, vorzugsweise $C_1$-$C_5$-Alkyliden, z.B. Methylen, Aethyliden, 1,1-Propyliden oder 1,1-(3-Methyl)-butyliden (Isopentyliden), kann aber auch 1,1-Butyliden, 2,2-Propyliden (Isopropyliden) oder 1,1-(2-Methyl)-propyliden (Isobutyliden) sein.

Säureadditionssalze von Verbindungen der Formel I sind beispielsweise pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten organischen Carbon- oder Sulfonsäuren, wie gegebenenfalls hydroxylierten aliphatischen Mono-oder Dicarbonsäuren, z.B. Acetate, Oxalate, Succinate, Fumarate, Maleinate, Maleate, Ascorbinate oder Citrate, der aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die

deshalb bevorzugt sind.

Die Verbindungen der Formel I und ihre pharmazeutisch verwendbaren Salze besitzen wertvolle pharmakologische, insbesondere nootrope Eigenschaften. So vermindern sie an der Maus in Dosen ab etwa 0,1 mg/kg i.p. sowie p.o. die amnesiogene Wirkung eines Elektroschocks in mindestens gleichem Ausmass wie nach Verabfolgung einer nootrop wirksamen Dosis von Piracetam (100 mg/kg i.p.). Zum Nachweis der nootropen Wirkung kann beispielsweise der Two-Compartment-Test herangezogen werden.

Als Literatur über pharmakologische Modelle dieser Art seien z.B. genannt: S.J. Sara und D. Lefevre, Psychopharmacologia 25, 32-40 (1972), Hypoxia-induced amnesia in one-trial learning and pharmacological protection by piracetam. Boggan, W.O., und Schlesinger, K., in Behavioral Biology 12, 127-134 (1974).

Im weiteren zeigen die Verbindungen der Formel I eine starke gedächtnisverbessernde Wirkung im Step-down Passive Avoidance Test nach Mondadori und Waser, Psychopharmacology 63, 297-300 (1979). Die Substanzen sind wirksam bei intraperitonealer Gabe 30 Minuten vor dem Lernversuch (wirksame Dosen 0,1, 1, 10 mg/kg). Eine deutliche Wirkung war auch feststellbar bei peroraler Applikation 60 Minuten vor dem Lernversuch (wirksame Dosen 0,1, 1, 10 mg/kg) sowie bei intraperitonealer Applikation unmittelbar nach dem Lernversuch (wirksame Dosen 0,1, 1, 10 mg/kg).

Die Verbindungen der Formel I und ihre pharmazeutisch verwendbaren Salze können dementsprechend als Nootropika, beispielsweise zur Behandlung zerebraler Leistungsinsuffizienz, insbesondere von Gedächtnisstörungen unterschiedlicher Genese, wie senile Demenz oder Demenz vom Alzheimer-Typ, ferner von Folgezuständen von Hirntraumata und Apoplexien, Verwendung finden.

In den Referenzen EP-A-089 900 und FR-A-2 382 441 werden nootrope Wirkstoffe beschrieben, die sich strukturell von denen der Formel I unterscheiden.

Die Erfindung betrifft in erster Linie Verbindungen der Formel I, worin $R_1$ einen unsubstituierten oder durch $C_1$-$C_4$-Alkyl, $C_1$-$C_4$-Alkoxy, Halogen der Atomnummer bis und mit 35 und/oder Trifluormethyl mono- oder disubstituierten Phenyl- oder Naphthylrest bedeutet, $X_1$ $C_1$-$C_7$-Alkyliden darstellt, $X_2$ Methylen, Aethylen oder Oxoäthylen bedeutet und $R_2$ Wasserstoff, $C_1$-$C_7$-Alkyl oder eine Gruppe der Formel Ia bedeutet, in der $X_4$ $C_1$-$C_7$-Alkyliden und $R_3$ Wasserstoff oder einen unsubstituierten oder durch $C_1$-$C_4$-Alkyl, $C_1$-$C_4$-Alkoxy, Halogen der Atomnummer bis und mit 35 und/oder Trifluormethyl mono- oder disubstituierten Phenyl- oder Naphthylrest darstellt, und ihre Salze, insbesondere pharmazeutisch verwendbaren Salze.

Die Erfindung betrifft vor allem Verbindungen der Formel I, worin $R_1$ unsubstituiertes oder durch Halogen der Atomnummer bis und mit 35, wie Chlor, $C_1$-$C_4$-Alkoxy, wie Methoxy, $C_1$-$C_4$-Alkyl, wie Methyl, oder Trifluormethyl substituiertes Phenyl oder unsubstituiertes Naphthyl bedeutet, $X_1$ terminal gebundenes $C_1$-$C_7$-Alkyliden, wie Methylen, Isobutyliden oder Isopentyliden, bedeutet, $X_2$ Oxoäthylen darstellt und $R_2$ Wasserstoff darstellt, und ihre Salze, insbesondere pharmazeutisch verwendbaren Salze.

Die Erfindung betrifft insbesondere Verbindungen der Formel I, worin $R_1$ unsubstituiertes oder durch Halogen der Atomnummer bis und mit 35, wie Chlor, $C_1$-$C_4$-Alkoxy, wie Methoxy, $C_1$-$C_4$-Alkyl, wie Methyl, oder Trifluormethyl substituiertes Phenyl oder unsubstituiertes Naphthyl bedeutet, $X_1$ terminal gebundenes $C_1$-$C_4$-Alkyliden, wie Methylen oder Isobutyliden, bedeutet, $X_2$ über die Carbonylgruppe mit der Teilstruktur -N($R_2$)- verbundenes Oxoäthylen darstellt und $R_2$ Wasserstoff darstellt, und ihre Salze, insbesondere pharmazeutisch verwendbaren Salze.

Die Erfindung betrifft ebenso insbesondere Verbindungen der Formel I, worin $R_1$ unsubstituiertes oder durch Halogen der Atomnummer bis und mit 35, wie Chlor, $C_1$-$C_4$-Alkoxy, wie Methoxy, $C_1$-$C_4$-Alkyl, wie Methyl, oder Trifluormethyl substituiertes Phenyl oder unsubstituiertes Naphthyl bedeutet, $X_1$ terminal gebundenes $C_1$-$C_7$-Alkyliden, wie Methylen oder Isopentyliden, oder vorzugsweise terminal gebundenes $C_1$-$C_4$-Alkyliden, wie Methylen, bedeutet, $X_2$ Methylen oder Aethylen darstellt und $R_2$ $C_1$-$C_4$-Alkyl, wie Methyl, oder eine Gruppe der Formel Ia bedeutet, in der $X_4$ terminal gebundenes $C_1$-$C_4$-Alkyliden, wie Methylen, ist und $R_3$ Wasserstoff bedeutet, und ihre Salze, insbesondere pharmazeutisch verwendbaren Salze.

Die Erfindung betrifft ganz besonders Verbindungen der Formel I, worin $R_1$ unsubstituiertes oder durch Halogen der Atomnummer bis und mit 35, wie Chlor,substituiertes Phenyl oder unsubstituiertes Naphthyl bedeutet, $X_1$ terminal gebundenes $C_1$-$C_4$-Alkyliden, wie Methylen, bedeutet, $X_2$ für über die Carbonylgruppe mit der Teilstruktur -N($R_2$)- verbundenes Oxoäthylen steht und $R_2$ Wasserstoff bedeutet, und ihre Salze, insbesondere pharmazeutisch verwendbare Salze.

Die Erfindung betrifft in allererster Linie Verbindungen der Formel I, worin $R_1$ unsubstituiertes oder durch Halogen der Atomnummer bis und mit 35, wie Chlor,substituiertes Phenyl oder unsubstituiertes Naphthyl bedeutet, $X_1$ terminal gebundenes $C_1$-$C_7$-Alkyliden, wie Methylen, Isobutyliden oder Isopentyliden, darstellt, $X_2$ über die Carbonylgruppe mit der Teilstruktur -N($R_2$)- verbundenes Oxoäthylen und $R_2$ Wasserstoff bedeutet, und ihre Salze, insbesondere pharmazeutisch verwendbare Salze.

Die Erfindung betrifft namentlich die in den Beispielen genannten Verbindungen der Formel I und ihre Salze, insbesondere pharmazeutisch verwendbaren Salze.

Die Erfindung betrifft ferner ein auf an sich bekannten Methoden beruhendes Verfahren zur Herstellung von Verbindungen der Formel I und ihrer Salze. Dieses ist dadurch gekennzeichnet, dass man

a) Verbindungen der Formeln

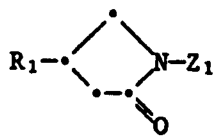 (II) und 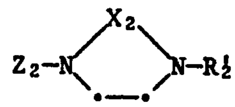 (III)

worin $Z_1$ eine Gruppe der Formel $-X_1-C(=O)-Z_3$ (IIa) und $Z_2$ Wasserstoff oder $Z_1$ Wasserstoff und $Z_2$ eine Gruppe der Formel $-C(=O)-X_1-Z_4$ (IIIa) darstellt, wobei $Z_3$ und $Z_4$ jeweils abspaltbare Reste bedeuten und $R_2'$ einen Rest $R_2$ oder eine Aminoschutzgruppe darstellt, oder deren Salze miteinander kondensiert oder

b) eine Verbindung der Formel

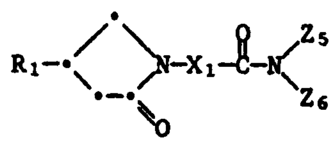 (IV),

worin $Z_5$ eine Grupee der Formeln $-CH_2-CH_2-N(R_2')-CH_2-Z_4$ (IVa), $-CH_2-CH_2-N(R_2')-CH_2CH_2-Z_4$ (IVb), $-CH_2-CH_2-N(R_2')-C(=O)-CH_2-Z_4$ (IVc) oder $-CH_2-CH_2-N(R_2')-CH_2-C(=O)-Z_3$ (IVd) und $Z_6$ Wasserstoff bedeutet oder $Z_5$ eine Gruppe der Formel $-X_2-N(R_2')-H$ (IVe) und $Z_6$ eine Gruppe der Formel $-CH_2CH_2-Z_4$ (IVg) bzw. $Z_5$ eine Gruppe der Formel $-CH_2CH_2-N(R_2')-H$ (IVh) und $Z_6$ eine Gruppe der Formel $-CH_2-Z_4$ (IVi), $-CH_2CH_2-Z_4$ (IVg), $-C(=O)-CH_2-Z_4$ (IVj) oder $-CH_2-C(=O)-Z_3$ (IVk) darstellt oder $Z_5$ für eine Gruppe der Formel $-CH_2-CN$ (IVl) und $Z_6$ für eine Gruppe der Formel $-CH_2CH_2-OH$ (IVm) bzw. $-C(=O)-CH_2-OH$ (IVn) steht, oder deren Salze oder eine Verbindung der Formel

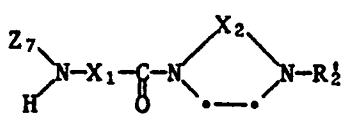 (V)

worin $Z_7$ eine Gruppe der Formeln

$-CH_2-CH(R_1)-CH_2-C(=O)-Z_3$ (Va) oder
$-C(=O)-CH_2-CH(R_1)-CH_2-Z_4$ (Vb)

bedeutet, oder deren Salze intramolekular cyclisiert, wobei $Z_3$ bzw. $Z_4$ jeweils abspaltbare Reste bedeuten und $R_2'$ einen Rest $R_2$ oder eine Aminoschutzgruppe darstellt, oder

c) Verbindungen der Formeln $R_1-CH(CH_2Z_4)-CH_2-C(=O)-Z_3$ (VIa) bzw.

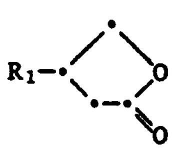 (VIb) und 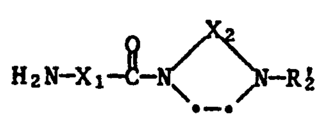 (VII),

miteinander kondensiert und jeweils eine gegebenenfalls vorhandene Aminoschutzgruppe $R_2'$ abspaltet, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I überführt, ein verfahrensgemäss erhältliches

Enantiomeren- oder Diastereomerengemisch in die Komponenten aufspaltet und/oder eine verfahrensgemäss erhältliche freie Verbindung in ein Salz oder ein verfahrensgemäss erhältliches Salz in die freie Verbindung oder in ein anderes Salz überführt.

Abspaltbare Reste $Z_3$ bzw. $Z_4$ sind beispielsweise reaktionsfähige veresterte Hydroxygruppen, abspaltbare Reste $Z_3$ ferner freie oder verätherte Hydroxygruppen oder in zweiter Linie Amino- oder Ammoniogruppen.

Als reaktionsfähige veresterte Hydroxygruppen kommen beispielsweise Halogenatome, im Falle von $Z_4$ auch Sulfonyloxygruppen in Betracht. Halogen $Z_3$ ist beispielsweise Chlor, Halogen $Z_4$ beispielsweise Chlor, Brom oder Jod. Sulfonyloxy $Z_4$ ist beispielsweise von einer organischen Sulfonsäure oder einer Halogensulfonsäure abgeleitetes Sulfonyloxy, wie Niederalkansulfonyloxy, z.B. Methan- oder Aethansulfonyloxy, gegebenenfalls substituiertes Benzolsulfonyloxy, z.B. Benzol-, p-Brombenzol- oder p-Toluolsulfonyloxy, oder Fluorsulfonyloxy.

Veräthertes Hydroxy $Z_3$ ist beispielsweise mit N-Hydroxysuccinimid oder einem aliphatischen, araliphatischen oder aromatischen Alkohol veräthertes Hydroxy, wie 1-(2,5-Dioxo)pyrrolidinoxy, Niederalkoxy, z.B. Methoxy, Aethoxy, Isopropyloxy oder Tertiärbutyloxy, Phenylniederalkoxy, z.B. Benzyloxy, oder gegebenenfalls substituiertes Phenoxy, z.B. Phenoxy, Pentachlorphenoxy, p-Nitrophenoxy oder 2,4-Dinitrophenoxy.

Als Aminogruppen $Z_3$ kommen primäre, sekundäre und tertiäre Aminogruppen, wie Amino, Mono- oder Diniederalkylamino, z.B. Methylamino, Aethylamino oder Dimethylamino, Niederalkylenamino bzw. Aza-, Oxa-oder Thianiederalkylenamino, z.B. 1-Pyrrolidinyl, 1-Piperidinyl oder 1-Morpholinyl, oder gegebenenfalls substituiertes Anilino, z.B. Anilino, p-Nitranilino oder 2,4-Dinitranilino ferner 1-Imidazolylgruppen, in Betracht.

Ammoniogruppen $Z_3$ sind beispielsweise quaternäre Ammoniogruppen, wie Triniederalkylammonio, z.B. Trimethylammonio, Triäthylammonio oder N-Isopropyl-N,N-dimethyl-ammonio, oder von aromatischen Stickstoffbasen abgeleitete quaternäre Ammoniogruppen, wie Pyridinio.

Salze der genannten Ausgangsstoffe und Zwischenprodukte sind beispielsweise Metall-, wie Alkalimetall-, z.B. Natrium-, Kalium-oder Lithiumsalze, von Verbindungen II ($Z_1$ = H oder $Z_3$ = OH), III ($Z_2$ = H) oder IV ($Z_5$ = IVd) oder Säureadditionssalze, z.B. Hydrochloride oder Hydrobromide, von Verbindungen III ($Z_2$ = H) oder IV ($Z_5$ = IVa, IVb, IVc, IVd; $Z_6$ = H oder $Z_5$ = IVh; $Z_6$ = IVg, IVi, IVj, IVk oder $Z_5$ = IVl; $Z_6$ = IVm, IVn).

Geeignete Aminoschutzgruppen $R_2'$ sind beispielsweise gegebenenfalls substituierte $\alpha$-Aralkyl-, wie Benzyl-, oder Benzyloxycarbonylgruppen, veresterte oder veretherte Hydroxymethylgruppen, wie Pivaloyloxymethyl, Methoxymethyl, 2-Chlorethoxymethyl oder Benzyloxymethyl, Tetrahydropyranyl oder Triniederalkylsilyl, wie Trimethylsilyl. Die Schutzgruppe wird beispielsweise durch Umsetzung der zu schützenden Verbindung mit einem entsprechenden Halogenderivat bzw. mit Chlorjodmethan ($Cl-CH_2I$), einem Alkalimetall-, z.B. Natriumpivalat, -methanolat, -1,2-dichlorethanolat oder -benzylalkoholat und bzw. mit Dihydropyran, eingeführt. Geschütztes Amino ist dementsprechend beispielsweise Silylamino, wie Trimethylsilylamino, z.B. Trimethylsilylamino, kann aber auch Phenyl-, Diphenyl- oder Triphenylniederalkylamino, wie Benzylamino, Diphenylmethylamino oder Tritylamino sein.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Kondensation von Verbindungen II und III gemäss Verfahrensvariante a) sowie die Cyclisierung von Verbindungen IV bzw. V gemäss Verfahrensvariante b) erfolgt in üblicher Weise, ausgehend von Verbindungen II und III und von Verbindungen IV, worin $Z_3$ reaktionsfähiges verestertes oder veräthertes Hydroxy bzw. $Z_4$ reaktionsfähiges verestertes Hydroxy ist, beispielsweise in Gegenwart eines basischen Kondensationsmittels oder, indem man die Reaktionskomponente II bzw. III, worin $Z_1$ bzw. $Z_2$ Wasserstoff ist, oder eine Verbindung IV, worin $Z_5$ eine Gruppe der Formel $-N(R_2')-$ aufweist bzw. $Z_6$ Wasserstoff ist, in einer Metallsalzform einsetzt, ausgehend von Verbindungen IV, worin $Z_5$ eine Gruppe der Formel IVl und $Z_6$ eine Gruppe IVm bzw. IVn bedeutet, insbesondere in Gegenwart eines sauren Mittels, und ausgehend von Verbindungen II bzw. IV, worin $Z_3$ Hydroxy ist, beispielsweise unter Dehydratisierung des primär gebildeten Ammoniumsalzes, beispielsweise durch trockenes Erhitzen, z.B. auf etwa 60 bis etwa 180 °C, oder insbesondere Behandeln mit einem wasserbindenden Mittel.

Basische Kondensationsmittel sind beispielsweise Alkalimetallhydride, wie Natriumhydrid, Alkalimetallamide, wie Natriumamid, oder von sterisch gehinderten aliphatischen Sekundäraminen abgeleitete Alkalimetallamide, z.B. Lithium-N,N-diisopropyl-amid, ferner Alkalimetallniederalkanolate, wie Kaliumtertiärbutanolat, ferner Natriummethanolat.

Für die Umsetzung von Verbindungen II und III, worin $Z_1$ eine Gruppe $-X_1-C(=O)-Z_3$ (IIa), $Z_2$ Wasserstoff, $X_2$ in $\alpha$-Stellung zur Gruppe $Z_2$-N< keine Oxogruppe aufweist und $Z_3$ reaktionsfähiges verestertes Hydroxy ist, sowie für die Cyclisierung von Verbindungen IV, worin $Z_5$ eine Gruppe IVe, worin $X_2$ in $\alpha$-Stellung zur Gruppe $-N(R_2')-H$ keine Oxogruppe aufweist, und $Z_6$ eine Gruppe IVg bzw. $Z_5$ eine Gruppe IVe, in der $X_2$ für Methylen steht, und $Z_6$ eine Gruppe IVg bedeutet und $Z_4$ reaktionsfähiges verestertes Hydroxy ist, sind ferner Alkali- oder Erdalkalimetallhydroxide, wie Natrium- oder Kaliumhydroxid, sowie organische Stickstoffbasen, wie Triniederalkylamine, z.B. Triäthylamin, oder tertiäre aromatische Stickstoffbasen, z.B. Pyridin, geeignet.

Saure Kondensationsmittel sind beispielsweise starke Protonensäuren, wie Mineralsäuren, z.B. Schwefelsäure in Essigsäure oder Dibutyläther oder Chlorwasserstoffsäure in Diäthyl- oder Methyl-tertiärbutyläther.

Wasserbindende Mittel sind beispielsweise Carbodiimide, wie N,N'-Dicyclohexylcarbodiimid, oder Halogenide oder Ester der Kohlensäure oder insbesondere von Sauerstoffsäuren des Schwefels oder Phosphors, wie Diniederalkylcarbonate, bzw. -pyrocarbonate, z.B. Diäthylpyrocarbonat, Phosgen, Diniederalkylsulfite, z.B. Dimethylsulfit, oder Triniederalkylphosphite, z.B. Trimethylphosphit, oder vor allem gegebenenfalls substituierte Triphenylphosphite, z.B. Triphenylphosphit.

Ausgangsstoffe II können beispielsweise hergestellt werden, indem man einen entsprechenden 4-Amino-3-$R_1$-buttersäureniederalkylester, beispielsweise durch Behandeln mit Schwefelsäure oder äthanolischer Salzsäure, zu dem entsprechenden 4-$R_1$-Pyrrolidin-2-on (II; $Z_1$ = H) cyclisiert und zur Herstellung von Verbindungen II, worin $Z_1$ eine Gruppe IIa bedeutet, dieses in Gegenwart eines basischen Kondensationsmittels, z.B. von Natriummethanolat, mit einer Verbindung der Formel $Z_4-X_1-C(=O)-Z_3$ (VIII) umsetzt, wobei $Z_4$ Iod, Brom oder Chlor und $Z_3$ insbesondere Niederalkoxy, z.B. Methoxy, bedeutet, das Reaktionsprodukt (II; $Z_1$ = IIa; $Z_3$ = veräthertes Hydroxy) erforderlichenfalls zur entsprechenden Säure (II; $Z_1$ = IIa; $Z_3$ = Hydroxy) hydrolysiert, beispielsweise durch Behandeln mit Natronlauge, diese erforderlichenfalls, beispielsweise mittels Thionylchlorid, halogeniert und das Reaktionsprodukt (II; $Z_1$ = IIa; $Z_3$ = Halogen, z.B. Chlor) erforderlichenfalls mit Ammoniak oder einem Amin der Formel $Z_3$-H(IX) kondensiert.

Ausgangsstoffe III, worin $Z_2$ und $R_2'$ Wasserstoff bedeuten, sind sämtlich bekannt. Ausgangsstoffe III, worin $Z_2$ Wasserstoff und $R_2'$ Niederalkyl oder eine Gruppe der Formel Ia bedeutet, können z.B. durch Umsetzung eines Imino-di(essigsäure)diniederalkylesters bzw. Benzylimino-di(essigsäure)-diniederalkylesters mit einem Niederalkylamin bzw. mit einer Verbindung der Formel

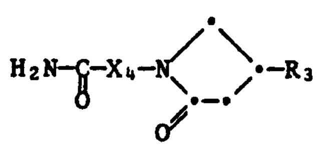

(XXX)

und gegebenenfalls hydrogenolytische Abspaltung der 4-Schutzgruppe bzw. durch Umsetzung von Imidazol, Piperazin oder Piperazin-2,5-dion mit einem reaktiven Ester eines Niederalkanols, wie einem Niederalkylhalogenid oder -p-toluol-sulfonat, bzw. einer Verbindung der Formel

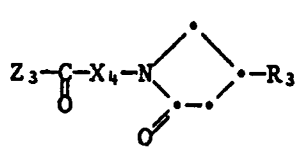

(XXXI),

worin $Z_3$ eine der angegebenen Bedeutungen hat und z.B. für Chlor steht, oder durch Kondensation einer Verbindung der Formel

$H_2N-CH_2CH_2-NH-R_3$ (XXXII)

mit Formaldehyd bzw. einem reaktiven Derivat davon, z.B. Dimethoxymethan, erhalten werden.

Ausgangsstoffe III, worin $Z_2$ eine Gruppe IIIa bedeutet, können ausgehend von den, z.B. wie vorstehenden entsprechenden Verbindungen III, worin $Z_2$ Wasserstoff ist, durch Kondensation mit Verbindungen der Formel $Z_4-X_1-C(=O)-Z_3$ (VIII; $Z_3$-Halogen) erhalten werden.

In einer bevorzugten Ausführungsform der Verfahrensvariante a) geht man beispielsweise von einer Verbindung der Formel II aus, worin $Z_1$ eine Gruppe der Formel $-X_1-C(=O)-Z_3$ (IIa; $Z_3$ = OH) bedeutet, und setzt diese in Gegenwart eines Phosphorigsäureesters, z.B. von Triphenylphosphit, mit einer Verbindung der Formel III um, worin $Z_2$ für Wasserstoff steht. Die Reaktionskomponente II wird dabei vorzugsweise durch Umsetzung des entsprechenden 4-$R_1$-pyrrolidin-2-ons mit einer entsprechenden Verbindung der Formel $Z_4-X_1-C(=O)-Z_3$ (VIII; $Z_4$ = Brom, $Z_3$ = Niederalkoxy, z.B. Methoxy) in Gegenwart eines Alkalimetallniederalkanolates, z.B. von Natriummethanolat, und anschliessende Hydrolyse des erhaltenen Esters (II; $Z_1$ = IIa, $Z_3$ = Niederalkoxy, z.B. Methoxy), beispielsweise durch Behandeln mit Natronlauge, und anschliessende Säurebehandlung, erhalten.

In einer anderen bevorzugten Ausführungsform der Verfahrensvariante a) setzt man eine Verbindung II, worin $Z_1$ Wasserstoff ist, in Gegenwart eine Alkalimetallhydrides, z.B. von Natriumhydrid in Dioxan, mit einer Verbindung III um, worin $Z_2$ eine Gruppe der Formel IIIa ($Z_4$ = Chlor oder Brom) darstellt.

Ausgangsstoffe IV, worin $Z_5$ eine Gruppe IVa, IVb, IVc bzw. IVd und $Z_6$ Wasserstoff oder $Z_5$ eine Gruppe IVe und $Z_6$ eine Gruppe der Formel IVg bedeutet, werden vorzugsweise in situ hergestellt und ohne Isolierung cyclisiert, beispielsweise indem man eine Verbindung der Formel

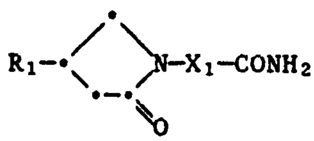

(XI)

mit einer Verbindung der Formeln

$Z_4-CH_2-N(R_2')-CH_2CH_2-Z_4$ (XIIa),
$Z_4-CH_2CH_2-N(R_2')-CH_2CH_2-Z_4$ (XIIb),
$Z_4-CH_2-C(=O)-N(R_2')-CH_2CH_2-Z_4$ (XIIc) bzw.
$Z_3-C(=O)-CH_2-N(R_2')-CH_2CH_2-Z_4$ (XIId) umsetzt

oder zunächst mit einer Verbindung der Formeln

$Z_4-CH_2CH_2-N(R_2')H$ (XIIIa),
$Z_3-C(=O)-CH_2-N(R_2')-H$ (XIIIb),
$Z_4-CH-C(=O)-N(R_2')-H$ (XIIIc) bzw.
$Z_4-CH_2-N(R_2')-H$ (XIIId)

und anschliessend mit einer Verbindung der Formel

$Z_4-CH_2CH_2-Z_4$ (XIVb)

umsetzt. Man kann aber auch eine Verbindung der Formel

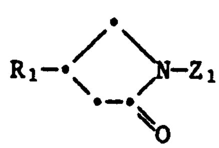

(II),

worin $Z_1$ eine Gruppe der Formel

$-X_1-C(=O)-Z_3$ (IIa)

bedeutet, mit einer Verbindung der Formeln

$H_2N-X_2-N(R_2')-CH_2CH_2-Z_4$ (XVa ) oder

zunächst mit einer Verbindung der Formel

$H_2N-X_2-N(R_2')-H$ (XVIa)

und anschliessend mit einer Verbindung XIVb bzw. zunächst mit einer Verbindung der Formel

$H_2N-CH_2CH_2-N(R_2')-H$ (XVIb)

und anschliessend mit einer Verbindung der Formel

$Z_4-CH_2-C(=O)-Z_3$ (XVIIa) bzw. XIVb

umsetzen.

Ausgangsstoffe IV, worin $Z_5$ eine Gruppe IVl und $Z_6$ eine Gruppe IVm bzw. IVn bedeutet, werden z.B. erhalten, indem man Aminoacetonitril mit Aethylenoxid umsetzt, in dem erhaltenen 2-(2-Hydroxyäthylamino)acetonitrildie Hydroxygruppe erforderlichenfalls, z.B. durch Acetylieren, schützt, das Reaktionsprodukt mit einer Verbindung II, worin $Z_1$ eine Gruppe der Formel $-X_1-C(=O)-Z_3$ (IIa) bedeutet, kondensiert und erforderlichenfalls die Schutzgruppe abspaltet.

Die vorstehenden Umsetzungen werden vorzugsweise in Gegenwart eines der genannten basischen Kondensationsmittel vorgenommen. Ebenso haben $Z_3$ und $Z_4$ jeweils die vorstehend angegebenen Bedeutungen, wobei $Z_3$ insbesondere für Niederalkoxy oder gegebenenfalls substituiertes Phenoxy und $Z_4$ insbesondere für Chlor, Brom oder Jod oder eine Sulfonyloxygruppe, z.B. p-Toluolsulfonyloxy steht.

Auch Zwischenprodukte V werden vorzugsweise in situ hergestellt und ohne Isolierung cyclisiert, beispielsweise indem man eine Verbindung der Formel

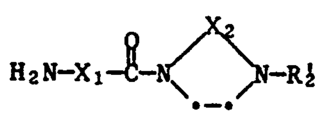

(VII)

unter basischen Bedingungen mit einer Verbindung der Formel

$Z_4-CH_2-CH(R_1)-CH_2-C(=O)-Z_3$ (VIa)

oder mit einer Verbindung der Formel

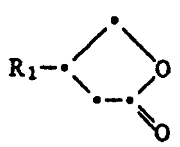

(VIb)

umsetzt. Dabei wird intermediär ein Zwischenprodukt V gebildet, worin $Z_7$ im erstgenannten Fall eine Gruppe Va oder Vb und im zweitgenannten Fall eine Gruppe Vb ($Z_4$ = Hydroxy) bedeutet, die erfindungsgemäss weiterreagiert.

In einer bevorzugten Ausführungsform der Verfahrensvariante b) setzt man ein 4-$R_1$-Pyrrolidin-2-on (II; $Z_1$ = H) in Gegenwart eines Alkalimetallalkanolates oder -hydrides, z.B. von Natriummethanolat oder Natriumhydrid, mit einer Verbindung der Formel

$Z_4-X_1-C(=O)-N(H)-CH_2CH_2-N(R_2')-CH_2CH_2-Z_4$ (XXIVa)
$Z_4-X_1-C(=O)-NH-CH_2CH_2-N(R_2')-CH_2-Z_4$ (XXIVb)
$Z_4-X_1-C(=O)-NH-CH_2CH_2-N(R_2')-CH_2-C(=O)-Z_3$ (XXIVc ) bzw.
$Z_4-X_1-C(=O)-NH-CH_2CH_2-N(R_2')-C(=O)-CH_2-Z_4$ (XXIVd)

oder zunächst mit einer Verbindung der Formel
$Z_4-X_1-C(=O)-Z_3$ (VIII; $Z_4$ = Chlor oder Brom, $Z_3$ = Amino)

und anschliessend mit einer Verbindung der Formel $Z_4$-$CH_2$-$N(R_2')$-$CH_2CH_2$-$Z_4$ (XXVa), $Z_4$-$CH_2CH_2$-$N(R_2')$-$CH_2CH_2$-$Z_4$ (XXVb), $Z_4$-$CH_2$-C(=O)-N)($R_2'$)-$CH_2$-$CH_2$-$Z_4$ (XXVc) oder $Z_3$-C(=O)-$CH_2$-$N(R_2')$-$CH_2CH_2$-$Z_4$ (XXVc) um. Dabei wird intermediär ein Zwischenprodukt IV gebildet, worin $Z_5$ eine Gruppe IVa, IVb, IVc bzw. IVd und $Z_6$ Wasserstoff bedeutet, das isoliert werden kann, wenn man die Umsetzung in Gegenwart eines Alkalimetallalkoholates vornimmt, bzw. erfindungsgemäss weiterreagiert, wenn man die Umsetzung in Gegenwart eines Alkalimetallhydrides vornimmt.

Zu einer anderen bevorzugten Ausführungsform der Verfahrensvariante b) behandelt man eine Verbindung der Formel IV ($Z_5$ = IVl, $Z_6$ = IVm bzw. IVn), erhältlich z.B. durch Umsetzung einer Verbindung (II; $Z_1$ = IIa, $Z_3$ = Amino) zunächst mit 2-Jodethanol oder einem Glykolsäureniederalkylester und anschliessend mit Chloracetonitril, in Analogie zur bekannten Ritter-Reaktion mit einem sauren Kondensationsmittel, z.B. mit Schwefelsäure.

In einer weiteren bevorzugten Ausführungsform der Verfahrensvariante b) setzt man eine Verbindung III ($Z_2$ = IIIa; $Z_4$ = Halogen) mit einer Säure der Formel $H_2N$-$CH_2$-$CH(R_1)$-$CH_2$-C(=O)-$Z_3$ (XXVI; $Z_3$ = OH) oder ein reaktionsfähiges Carboxyderivat, wie dem Halogenid oder einem Ester, z.B. einem Niederalkyl- oder gegebenenfalls substituierten, wie p-nitrierten, Phenylester, davon um. Dabei wird intermediär ein Zwischenprodukt V ($Z_7$ = Va) gebildet, das unter den erwähnten basischen Bedingungen erfindungsgemäss cyclisiert.

Die Umsetzung von Verbindungen VIa bzw. VIb gemäss Verfahrensvariante c) erfolgt in üblicher Weise, ausgehend von Verbindungen VIa beispielsweise unter neutralen oder basischen Bedingungen, d.h. in einem inerten Lösungsmittel, erforderlich unter Erwärmen, und ausgehend von Verbindungen VIb beispielsweise in Gegenwart eines sauren Kondensationsmittels. Basische Kondensationsmittel sind beispielsweise Alkalimetallhydride, wie Natriumhydrid, Alkalimetallamide, wie Natriumamid, oder von sterisch gehinderten aliphatischen Sekundäraminen abgeleitet Alkalimetallamide, z.B. Lithium-N,N-diisopropyl-amid, ferner Alkalimetallniederalkanolate, wie Kaliumtertiärbutanolat, ferner Natriummethanolat, ferner Alkali-oder Erdalkalimetallhydroxide, wie Natrium- oder Kaliumhydroxid, sowie organische Stickstoffbasen, wie Triniederalkylamine, z.B. Triäthylamin, oder tertiäre aromatische Stickstoffbasen, z.B. Pyridin. Saure Kondensationsmittel sind beispielsweise starke Protonensäuren, wie Mineralsäuren, z.B. Schwefelsäure oder Chlorwasserstoffsäure.

Ausgangsstoffe VIa bzw. VIb werden beispielsweise hergestellt, indem man eine Verbindung der Formel $R_1$-$CH_2$-CH=O (XXI) nach Metallierung in $\alpha$-Stellung, z.B. durch Behandeln mit einem Alkalimetallhydrid oder einem Alkalimetallniederalkanolat, mit einer Verbindung der Formel $Z_4$-$CH_2$-C(=O)-$Z_3$ (XXII; $Z_3$ = Niederalkoxy; $Z_4$ = Halogen) umsetzt und das Reaktionsprodukt mit einem für die Reduktion der Formylgruppe zu Hydroxymethyl geeigneten Reduktionsmittel, z.B. mit Natriumcyanoborhydrid, behandelt. Der gebildete Hydroxyester VIa kann dann, erforderlichenfalls nach Vorbehandlung mit einem Halogenierungsmittel, wie Phosphortribromid und Pyridin, unter neutralen oder basischen Bedingungen mit der Reaktionskomponente umgesetzt oder unter sauren Bedingungen zur entsprechenden Verbindung VIb cyclisiert werden.

Ausgangsstoffe VII werden beispielsweise erhalten, indem man Verbindungen der Formeln $H_2N$-$X_1$-C(=O)-$Z_3$ (XXIII; $Z_3$ = Niederalkoxy, gegebenenfalls substituiertes Phenoxy) und

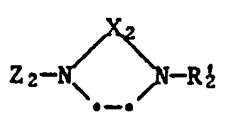

(III; $Z_2$ = H)

miteinander kondensiert.

Die Freisetzung intermediär geschützter Reste, z.B. Abspaltung einer Aminoschutzgruppe $R_2'$, erfolgt in üblicher Weise, beispielsweise durch Hydrogenolyse, z.B. in Gegenwart von Platin- oder Palladiumkatalysatoren, bzw. Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salz- oder Essigsäure.

Verfahrensgemäss erhältliche Verbindungen können in üblicher Weise in andere Verbindungen der Formel I überführt werden.

So kann man beispielsweise in den Rest $R_1$ von Verbindungen Substituenten einführen, Niederalkyl beispielsweise durch Umsetzung mit einem Niederalkylhalogenid in Gegenwart von Aluminiumtrichlorid, Niederalkoxy beispielsweise durch Nitrieren, Reduktion der Nitrogruppe zur Aminogruppe, Diazotieren derselben und Behandeln des gebildeten Diazoniumsalzes mit dem entsprechenden Niederalkanol unter

Erwärmen und Halogen beispielsweise durch Behandeln mit Chlor oder Brom, vorteilhaft in Gegenwart einer Lewissäure, z.B. von Eisen-III-chlorid. Man kann aber auch Halogen durch Trifluormethyl ersetzen, beispielsweise durch Behandeln mit Trifluorjodmethan in Gegenwart von Kupferpulver oder Kupfer-I-jodid.

Ferner kann man Verbindungen der Formel I, worin $R_2$ Wasserstoff ist, durch einen von Wasserstoff verschiedenen Rest $R_2$ substituieren, beispielsweise durch Umsetzung mit einem Niederalkylhalogenid oder einer Verbindung der Formel

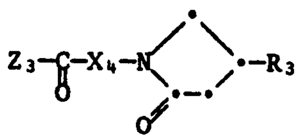

(XXXI),

worin $Z_3$ eine der angegebenen Bedeutungen hat und z.B. für Chlor steht, in Gegenwart einer für die Verfahrensvariante a) und b) angegebenen Base, wie Natriumhydrid. Umgekehrt kann man Niederalkyl $R_2$ durch Wasserstoff ersetzen, z.B. durch Behandeln mit einem Halogenameisensäureniederalkylester und nachfolgende Hydrolyse.

Die neuen Verbindungen können, je nach Wahl der Ausgangsstoffe und Arbeitsweisen, in Form eines der möglichen Isomeren, z.B. je nach der Anzahl der asymmetrischen Kohlenstoffatome als optische Isomere, wie in Form eines Enantiomeren, wie Antipoden bzw. Diastereomeren oder als Gemische derselben, wie Enantiomerengemische, z.B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung einer Verbindung der Formel I, mit einer optisch aktiven Säure oder einem Anhydrid davon bzw. einem reaktionsfähigen Ester eines optisch aktiven Alkohols und Trennung des erhaltenen diastereomeren Esters, z.B. auf Grund ihrer verschiedenen Löslichkeiten, in die Diastereomeren, aus denen die Enantiomeren durch Einwirkung geeigneter Mittel freigesetzt werden können. Racemate Verbindungen der Formel I können auch durch Umsetzung mit einer optisch aktiven Hilfsverbindung in Diastereomerengemische, z.B. mit einer optisch aktiven Säure in Gemische diastereomerer Salze, und Trennung derselben in die Diastereomeren, aus denen die Enantiomeren in der jeweils üblichen Weise freigesetzt werden können, gespalten werden.

Für diesen Zweck übliche optisch aktive Säuren sind z.B. optische aktive Carbon- oder Sulfonsäuren, wie D- oder L-Weinsäure, Di-o-Toluylweinsäure, Aepfelsäure, Mandelsäure, Camphersulfonsäure oder Chinasäure.

Ferner können erhaltene freie salzbildende Verbindungen in an sich bekannter Weise in Salze überführt werden, z.B. durch Umsetzen einer Lösung der freien Verbindung in einem geeigneten Lösungsmittel oder Lösungsmittelgemisch mit einer entsprechenden Säure oder mit einem geeigneten Ionenaustauscher.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak.

Erhaltene Salze können in an sich bekannter Weise in andere Salze überführt werden, z.B. durch Behandlung eines Salzes einer organischen Säure mit einem geeigneten Metallsalz, wie einem Natrium-, Barium- oder Silbersalz, einer Säure in einem geeigneten Lösungsmittel, in welchen ein sich bildendes anorganisches Salz unlöslich ist und damit aus dem Reaktionsgemisch ausscheidet.

Die Verbindungen, einschliesslich ihre Salze, können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfinding betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

So können, wie erwähnt, Zwischenprodukte der Formeln IV und V gemäss der Verfahrensvariante b) in situ gebildet und ohne Isolierung weiterumgesetzt werden.

Neue Ausgangsstoffe, die speziell für die Herstellung der erfindungsgemässen Verbindungen entwickelt wurden, insbesondere die zu den eingangs als bevorzugt gekennzeichneten Verbindungen der Formeln I führende Ausgangsstoffauswahl, die Verfahren zu ihrer Herstellung sowie ihre Verwendung als Zwischenprodukte, bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Saccharose, Mannitol, Sorbitol, Cellulose und/oder Schmiermittel, z.B. Kieselerde, Talk, Stearinsäure oder Salze davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel Farbstoffe, Geschmackstoffe und Süssmittel aufweisen. Ferner kann man die neue Verbindung der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesonere von etwa 1 % bis etwa 50 %, Lyophilisate bis zu 100 % des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Gewicht von etwa 70 kg vorzugsweise zwischen etwa 20 mg und etwa 500 mg.

Die nachfolgenden Beispiele dienen zur Illustration der Erfindung; Temperaturen sind in Celciusgraden, Drucke in mbar angegeben.

Beispiel 1: 82,5 g (325 mMol) 2-[4-(p-Chlorphenyl)-2-oxo-pyrrolidin-1-y]essigsäure, 32,5 g (325 mMol) Piperazin-2-on (Ketopiperazin) und 100,8 g (325 mMol) frisch destilliertes Triphenylphosphit werden bei 180° verschmolzen. Man lässt auf 130° abkühlen und 5 Stunden stehen. Die erstarrte Reaktionsmasse wird auf Raumtemperatur abgekühlt und 1 Stunde mit 300 ml Dichlormethan verrührt. Man saugt ab, wäscht dreimal mit je 150 ml Dichlormethan nach und lässt an der Luft trocknen. Man erhält 1-[4-(p-Chlorphenyl)-2-oxo-pyrrolidin-1-ylacetyl]-3-oxo-piperazin vom Smp. 208-210°, das durch Auflösen in 200 ml heisser Essigsäure, Versetzen mit 800 ml Wasser und Kristallisation im Eisbad weiter gereinigt werden kann.

Das Ausgangsmaterial kann beispielsweise folgendermassen hergestellt werden:

13 g (565 mMol) Natrium werden portionsweise in 410 ml Aethanol eingetragen. Nach vollständiger Auflösung fügt man 107 g (545 mMol) 4-(p-Chlorphenyl)-2-oxo-pyrrolidin hinzu, lässt 2 Stunden bei Raumtemperatur rühren, dampft unter vermindertem Druck zur Trockne ein und trocknet über Nacht bei 100° unter vermindertem Druck. Das erhaltene Natrium-4-(p-chlorphenyl)-2-oxo-pyrrolidin wird in 340 ml Toluol aufgeschlämmt und unter Rühren bei 20 bis 25° tropfenweise mit einer Lösung von 63,7 ml (95,9 g; 574 mMol) Bromessigsäureäthylester versetzt. Man lässt 16 Stunden nachrühren, dampft unter vermindertem Druck bei etwa 70° zur Trockne ein und verteilt zwischen 300 ml Wasser und 600 ml Essigester. Die organische Phase wird abgetrennt, mit gesättigter Kochsalzlösung gewaschen, über Natriumsulfat getrocknet, filtriert und unter vermindertem Druck zur Trockne eingedampft. Man erhält 2-[4-(p-Chorphenyl)-2-oxo-pyrrolidin-1-yl]essigsäureäthylester, der zur Reinigung unter vermindertem Druck destilliert wird; Kp = 176-178° bei 0,02 Torr (0,027 mbar).

95,6 g (340 mMol) 2-[4-(p-Chlorphenyl)-2-oxo-pyrrolidin-1-yl]essigsäureäthylester werden in 440 ml Methanol gelöst, mit 22,5 g (40 mMol) Kaliumhydroxid (80 %ig) versetzt und 16 Stunden zum Rückfluss erhitzt. Man dampft unter vermindertem Druck bei 70° ein, versetzt mit 200 ml zu Salzsäure und schüttelt mit 800 ml Essigester aus. Die organische Phase wird mit gesättigter Kochsalzlösung gewaschen, auf 500

ml eingeengt und mit 200 ml Hexan versetzt. Der kristalline Niederschlag wird abgesaugt und getrocknet. Man erhält 2-[4-(p-Chlorphenyl)-2-oxo-pyrrolidin-1-yl]essigsäure vom Smp. 142-143°. Durch Eindampfen der Mutterlauge, Aufnehmen in 200 ml Essigester und Kristallisieren durch Hinzufügen von 100 ml Hexan kann weiteres Produkt gewonnen werden.

Beispiel 2: 22,4 g (44,6 mMol) 2-[4-(p-Chlorphenyl)-2-oxo-pyrrolidin-1-yl]-essigsäurepentachlorphenylester werden mit 4,46 g (44,6 mMol) Piperazin-2-on (Ketopiperazin) und 300 ml Dimethylformamid versetzt und 5 Stunden bei Raumtemperatur gerührt. Man dampft bei 70° unter vermindertem Druck zur Trockne ein, verrührt 1 Stunde mit 150 ml Essigester, versetzt mit 150 ml Diäthyläther, saugt ab, wäscht mit 50 ml Diäthyläther nach und lässt trocknen. Man erhält 1-[4-(p-Chlorphenyl)-2-oxo-pyrrolidin-1-ylacetyl]-3-oxo-piperazin vom Smp. 205-207°, das durch Umkristallisieren aus 300 ml Butanol, Waschen mit 50 ml Diäthyläther und Trocknen weiter gereinigt werden kann und dann bei 208-210° schmilzt.

Das Ausgangsmaterial kann z.B. folgendermassen hergestellt werden:

73,5 g (290 mMol) 2-[4-(p-Chlorphenyl)-2-oxo-pyrrolidin-1-yl)essigsäure werden in 900 ml Tetrahydrofuran suspendiert, mit 92,5 g (350 mMol) Pentachlorphenol versetzt und bei Raumtemperatur bis zur klaren Auflösung gerührt. Man kühlt im Eisbad ab, fügt innerhalb von 30 Minuten tropfenweise 65,8 g (319 mMol) Dicyclohexylcarbodiimid, gelöst in 180 ml Tetrahydrofuran, hinzu, lässt 1 Stunde im Eisbad und 16 Stunden bei Raumtemperatur nachrühren, filtriert von dem ausgefallenen Dicyclohexylharnstoff ab, dampft bei 60° unter vermindertem Druck zur Trockne ein und kristallisiert aus 400 ml Essigester um. Man erhält 2-[4-(p-Chlorphenyl)-2-oxo-pyrrolidin-1-yl]essigsäurepentachlorphenylester vom Smp. 135-136°. Durch Einengen der Mutterlauge kann weiteres Produkt gewonnen werden.

Beispiel 3: 10g (30 mMol) N-[4-(p-Fluorphenyl)-2-oxo-pyrrolidin-1-ylacetoxy]succinimid und 3,0 g (30 mMol) Piperazin-2-on (Ketopiperazin) werden in 100 ml Dimethylformamid 16 Stunden bei Raumtemperatur gerührt. Man dampft bei 70° unter vermindertem Druck zur Trockene ein, zieht in der Siedehitze mit Trichlormethan aus, lässt abkühlen, saugt ab und lässt an der Luft trocknen. Man erhält 1-[4-(p-Fluorphenyl)-2-oxo-pyrrolidin-1-ylacetyl]-3-oxo-piperazin vom Smp. 200-203°.

Das Ausgangsmaterial kann z.B. folgendermassen hergestellt werden:

Eine Lösung von 35,6 g (150 mMol) 2-[4-(p-Fluorphenyl)-2-oxo-pyrrolidin-1-yl]essigsäure und 17,2 g (150 mMol) N-Hydroxysuccinimid in 570 ml Dioxan wird portionsweise mit insgesamt 31,5 g (150 mMol) Dicyclohexylcarbodiimid versetzt, wobei die Temperatur der Reaktionsmischung durch Kühlen im Eisbad unter 30° gehalten werden soll. Nach Abklingen der exothermen Reaktion lässt man 16 Stunden bei Raumtemperatur nachrühren, filtriert von dem ausgeschiedenen Dicyclohexylharnstoff ab und dampft bei 70° unter vermindertem Druck zur Trockene ein. Der Rückstand wird in 200 ml Essigester gelöst und bis zur beginnenden Trübung mit Diäthyläther versetzt. Der alsbald ausfallende kristalline Niederschlag wird abgesaugt, mit Diäthyläther gewaschen und getrocknet. Man erhält N-[4-(p-Fluorphenyl)-2-oxo-pyrrolidin-1-yl-acetoxy]succinimid vom Smp. 120-123°.

Beispiel 4: Ein Gemisch von 10 g (3,7 mMol), 2-[4-(p-Chlorophenyl)-2-oxo-pyrrolidin-1-yl]-essigsäuremethylester und 10 g (100 mMol) N-Methylpiperazin wird bis zur Rotfärbung geschwenkt. Es wird unter vermindertem Druck bei 70° zur Trockene eingedampft. Der ölige Rückstand (14,3 g) wird in 100 ml n-Salzsäure gelöst und die Lösung mit 100 ml Aethyläther extrahiert. Die wässrige Phase stellt man mit konzentrierter Natronlauge alkalisch und extrahiert die so freigesetzte Base mit 500 ml Methylenchlorid. Nach Trocknen über Natriumsulfat, Filtrieren und Eindampfen erhält man 1-[4-(p-Chlorphenyl)-2-oxo-pyrrolidin-1-ylacetyl]-4-methyl-piperazin. Es zeigt im Massenspektrogramm ein Molekulargewicht von 336.

9,9 g der freien Base werden in 30 ml Aethanol gelöst, mit äthanolischer Salzsäure auf pH = 4 gestellt und das gebildete Hydrochlorid durch Zugabe von Aethyläther ausgefällt und abgesaugt. Man erhält 1-[4-(p-Chlorphenyl)-2-oxo-pyrrolidin-1-ylacetyl]-4-methyl-piperazin-bishydrochlorid vom Smp. 202 - 204°.

Beispiel 5: Eine Lösung von 15,8 g (50 mMol) N-[2-(2-Oxo-4-phenyl-pyrrolidin-1-yl)acetoxy]succinimid und 8,8 g (50 mMol) N-Benzylpiperazin in 100 ml Dimethylformamid wird 16 Stunden bei Raumtemperatur gerührt. Das Reaktionsgemisch wird unter vermindertem Druck bei 70° vom Lösungsmittel befreit. Der ölige Rückstand (28 g) wird in 100 ml Essigester aufgenommen, 3-mal mit je 50 ml Wasser gewaschen, mit gesättigter wässriger Kochsalzlösung ausgeschüttelt, über Natriumsulfat getrocknet, filtriert und auf 30 bis 40 ml eingeengt. Die wässrigen Auszüge enthalten N-Hydroxysuccinimid und werden verworfen. Die eingeengte Essigester-Schicht wird mit einer Lösung von 5,8 g Maleinsäure, gelöst in 100 ml warmem Essigester, versetzt. Das entstandene Salz fällt zuerst als Oel aus, kristallisiert aber beim Rühren im Eisbad durch. Nach Umkristallisieren aus Aethanol erhält man kristallines 1-[2-(2-Oxo-4-phenyl-pyrrolidin-1-yl)-acetyl]-4-benzyl-piperazin-maleinat vom Smp. 170-3°.

19 g des beschriebenen Salzes werden in 100 ml Wasser aufgeschlämmt, mit 100 ml Essigester überschichtet und mit gesättigter wässriger Natriumhydrogencarbonatlösung auf pH 8 bis 9 eingestellt. Die

Essigesterschicht wird mit gesättigter Kochsalzlösung gewaschen, über Natriumsulfat getrocknet, filtriert und zur Trockene eingedampft. Als Rückstand bleibt öliges 1-[2-(2-Oxo-4-phenyl-pyrrolidin-1-yl)acetyl]-4-benzylpiperazin von $R_f$ = 0,60; Laufmittel Toluol/Aethanol (1:1) zurück.
Die ölige Base wird in 300 ml Essigsäure aufgenommen und bei Anwesenheit von 1,5 g 5 %iger Palladiumkohle 16 Stunden bei 22° hydriert. Nach Aufnahme von 860 ml Wasserstoff wird vom Katalysator abfiltriert und das klare Filtrat unter vermindertem Druck bei 70° zur Trockene eingedampft. Der ölige Rückstand kristallisiert beim Digerieren mit Essigester im Eisbad und wird aus 200 ml Essigester umkristallisiert. Man erhält 1-[2-(2-Oxo-4-phenyl-pyrrolidin-1-yl)acetyl]piperazin-diacetat vom Smp. 121-124°.

Beispiel 6: Zu einer Suspension von 6,0 g (23,3 mMol) 1-[2-(2-Oxo-pyrrolidin-1-yl)acetyl]piperazin-hydrochlorid in 80 ml Methylenchlorid gibt man zuerst 2,6 g (3,6 ml; 25,5 mMol) Triäthylamin und dann tropfenweise eine Lösung von 13,7 (23,3 mMol) 2-[4-(p-Chlorphenyl-2-oxo-pyrrolidin-1-yl]-essigsäurepentachlorphenylester in 30 ml Methylenchlorid hinzu. Nach 16 Stunden Rühren bei 20° wird die Suspension abgesaugt. Man gewinnt 6,16 g einer weissen kristallinen Masse. In das Filtrat wird 110 ml Aethyläther eingerührt, wodurch weitere 6,51 g Niederschlag ausgefällt und gesammelt werden können. Beide Anteile werden vereinigt unter 200 ml Aethylether verrieben, abgesaugt, das erhaltene Produkt in 150 ml heissem Eisessig gelöst und bis zur Trübung mit warmem Wasser (50°) versetzt. Es wird auf 20° abgekühlt; nach 3 Stunden wird das ausgefallene kristalline Produkt abgesaugt und am Wasserbad getrocknet. Man erhält 1-[4-(p-Chlorphenyl)-2-oxo-pyrrolidin-1-ylacetyl]-4-(2-oxo-pyrrolidin-1-ylacetyl)-piperazin vom Smp. 224-226°.

Das Ausgangsmaterial kann z.B. wie folgt hergestellt werden: Eine Lösung von 14,4 g (60 mMol) N-[2-(2-Oxo-pyrrolidin-1-yl)acetozy]succinimid und 10,6 g (60 mMol) 1-Benzylpiperazin in 125 ml Dimethylformamid wird bei 20° 16 Stunden gerührt. Das Reaktionsgemisch wird unter vermindertem Druck 90° zur Trockene eingedampft. Der ölige Rückstand wird in 250 ml Wasser aufgenommen, mit einer Lösung von 5,4 g Oxalsäure in 5 ml Wasser versetzt und dann im Eisbad gerührt. Das ausgefallene, kristalline Oxalat wird abgesaugt, in 400 ml Wasser gelöst und mit konzentrierter Ammoniaklösung auf pH=9 gestellt. Die klare wässrige Lösung wird 2-mal mit je 30 ml Aether gewaschen, die ätherischen Auszüge werden verworfen und die wässrige Schicht unter vermindertem Druck bei 70° eingedampft. Der Rückstand besteht aus einer klebrigen Masse, die in 400 ml Aethanol bei 30° verrührt und vom Unlöslichen abgesaugt wird. Nach Eindampfen des klaren äthanolischen Filtrats erhält man ein leicht gelbliches Oel. Dieses wird in 130 ml Essigester aufgekocht, heiss filtriert und das Filtrat im Eisbad ausgerührt. Die ausgeschiedenen Kristalle werden abgesaugt und am Wasserbad getrocknet. Man erhält 1-[2-(2-Oxopyrrolidin-1-yl)acetyl])-4-benzyl-piperazin.

Eine Lösung von 13,2 g dieses Produktes in 300 ml Eisessig wird bei Anwesenheit von 1,5 g 5 %iger Palladiumkohle 4 Stunden bei 20° hydriert. Nach Aufnahme von 986 ml Wasserstoff wird die Hydrierung abgebrochen, vom Katalysator abgesaugt und das Filtrat unter vermindertem Druck bei 70° zur Trockene eingedampft. Der ölige Rückstand wird in 200 ml Essigester gelöst und mit alkoholischer Salzsäure auf pH=2 gestellt. Das aufgefallene Hydrochlorid wird abgenutscht und aus Aethanol umkristallisiert. Man erhält 1-[2-(2-Oxopyrrolidin-1-yl)acetyl]piperazin-hydrochlorid vom Smp. 232-234°. Das analog herstellbare Maleinat schmilzt bei 163-165°.

Beispiel 7: Ein Gemisch von 5,1 g (20,1 mMol) 2-[4-(p-Chlorphenyl)-2-oxo-pyrrolidin-1-yl]essigsäure, 3,5 g (20,1 mMol) 1-Benzylpiperazin, 6,57 g (21,1 mMol) Triphenylphosphit und 15 ml Dimethylformamid wird 3 Stunden bei 95 - 100°C gerührt. Dann wird das Gemisch unter vermindertem Druck bei 90° eingeengt bis zur Gewichtskonstanz. Der ölige Rückstand wird mit 40 ml Aceton verdünnt und mit einer Lösung von 2,3 g Maleinsäure in 20 ml Aceton versetzt. Das ausgeschiedene Maleat wird abgesaugt und mit wenig Aceton und Aethyläther gewaschen. Das erhaltene 1-[4-(p-Chlorphenyl)-2-oxo-pyrrolidin-1-ylacetyl]-4-benzyl-piperazin-maleinat vom Smp. 161-164°, wird aus Methanol und Aether umkristallisiert.

14,3 g der aus dem Maleat freigesetzten Base werden in 150 ml Eisessig mit Wasserstoff in Anwesenheit von 1,5 g 5 %iger Palladiumkohle katalytisch debenzyliert. Nach Aufnahme von 777 ml Wasserstoff wird vom Katalysator abgesaugt und das Filtrat unter vermindertem Druck bei 80° eingedampft. Der ölige Rückstand wird mit 60 ml Wasser und 40 ml n-Natronlauge versetzt und 2-mal mit je 50 ml Chloroform extrahiert. Nach Trocknen über Natriumsulfat, Filtrieren und Eindampfen erhält man 1-[4-(p-Chlorphenyl)-2-oxo-pyrrolidin-1-ylacetyl]piperazin als gelbliches Oel, das in 100 ml Aceton gelöst und durch Versetzen mit 3,4 g Maleinsäure in das Maleinat vom Smp. 148-150°C überführt wird.

Beispiel 8: 7,74 g (25 mMol) 2-[4-(p-Chlorphenyl)-2-oxo-pyrrolidin-1-yl]-4-methyl-pentansäure, 2,75 g (27,5 mMol) Piperazin-2-on und 0,3 g (2,5 mMol) 4-Methylaminopyridin werden in 100 ml Tetrahydrofuran gelöst und unter Rühren bei einer Temperatur von 10 bis 15° tropfenweise mit einer Lösung von 5,67 g (27,5 mMol) N,N′-Dicyclohexylcarbodiimid in 20 ml Tetrahydrofuran versetzt. Die Suspension lässt man 16 Stunden bei Raumtemperatur rühren.

Die Kristalle werden abgesaugt, das Filtrat unter vermindertem Druck eingedampft, der ölige Rückstand in 100 ml Methylenchlorid gelöst, 2-mal mit je 50 ml Salzsäure, 2-mal mit Natriumhydrogencarbonatlösung und 2-mal mit je 50 ml Wasser gewaschen. Die organische Phase wird abgetrennt, über Natriumsulfat getrocknet, filtriert und unter vermindertem Druck zur Trockne eingedampft.
Das Produkt wird mittels Flash-Säulenchromatographie gereinigt. Man erhält 1-{2-[4-(p-Chlorphenyl)-2-oxo-pyrrolidin-1-yl]-4-methyl-pentanoyl}-3-oxo-piperazin als Schaum.

Das Ausgangsmaterial kann z.B. folgendermassen hergestellt werden: 4,8 g (208 Mol) Natrium werden portionenweise in 150 ml Aethanol eingetragen. Nach vollständiger Auflösung fügt man 39,15 g (200 mMol) 4-(p-Chlorphenyl)-2-oxo-pyrrolidin hinzu, lässt 2 Stunden bei Raumtemperatur rühren, dampft unter vermindertem Druck zur Trockne ein, schlämmt 3-mal mit Toluol auf und dampft unter vermindertem Druck zur Trockne ein.
Das erhaltene 4-(p-Chlorphenyl)-2-oxo-pyrrolidon-natriumsalz wird in 200 ml Toluol aufgeschlämmt und unter Rühren bei 20 bis 25° tropfenweise mit einer Lösung von 40,2 g (191,3 mMol) 2-Brom-4-methyl-pentansäuremethylester in 50 ml Toluol versetzt.
Man lässt 16 Stunden nachrühren, dampft unter vermindertem Druck bei etwa 50 ° zur Trockne ein und verteilt zwischen 200 ml Wasser und 300 ml Essigester. Die organische Phase wird abgetrennt, mit gesättigter Kochsalzlösung gewaschen, über Natriumsulfat getrocknet, filtriert und unter vermindertem Druck zur Trockne eingedampft. Man erhält 2-[4-(p-Chlorphenyl)-2-oxo-pyrrolidin-1-yl]-4-methyl-pentansäuremethylester, der zur Reinigung unter vermindertem Druck im Kugelrohr destilliert wird. Kp = 200° bei 0,1 Torr.

53,3 g (164,6 mMol) 2-[4-(p-Chlorphenyl)-2-oxo-pyrrolidin-1-yl]-4-methyl-pentansäuremethylester werden in 230 ml Methanol gelöst, mit 10,9 g (164,4 mMol) Kaliumhydroxid (85 %ig) versetzt und 16 Stunden zum Rückfluss erhitzt. Man dampft unter vermindertem Druck bei 50° ein, versetzt mit 230 ml 2n-Salzsäure und verrührt die entstehende Suspension 1 Stunde bei 5°. Die Kristalle werden abgenutscht, mit Wasser gewaschen und unter vermindertem Druck bei 80° getrocknet. Durch Umkristallisieren aus Essigester/Hexan erhält man 46,1 g 2-[4-(p-Chlorphenyl)-2-oxo-pyrrolidin-1-yl]-4-methyl-pentansäure; Smp. 176-7°.

Beispiel 9: In analoger Weise wie in den Beispielen 1 bis 7 beschrieben kann man ferner 1-[4-(p-Chlorphenyl)-2-oxo-pyrrolidin-1-ylacetyl]-4-methyl-3-oxopiperazin herstellen.

Beispiel 10: 1,77 g 1-Chloracetyl-3-oxo-piperazin werden bei 80° in 500 ml Toluol suspendiert. Dann werden in 3 Portionen insgesamt 2,18 g 4-(p-Chlorphenyl)-2-oxo-pyrrolidin-2-on-natrium eingetragen. Man lässt 8 Stunden bei 80° rühren, dampft unter vermindertem Druck bei 70° zur Trockne ein und destilliert bei 150-155°/0,05 Torr ein Nebenprodukt ab, kocht den Rückstand mit Butanol aus, filtriert und dampft zur Trockne ein. Man erhält 1,5 g 1-[4-(p-Chlorphenyl)-2-oxo-pyrrolidin-1-ylacetyl]-3-oxo-piperazin vom Smp. 208-210°.

Beispiel 11: Tabletten, enthaltend je 50 mg 1-[4-(p-Chlorphenyl)-2-oxo-pyrrolidin-1-ylacetyl]-3-oxo-piperazin können wie folgt hergestellt werden:

| Zusammensetzung (1000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 12: Lacktabletten, enthaltend je 100 mg 1-[4-(p-Chlorphenyl)-2-oxo-pyrrolidin-1-ylacetyl]-3-oxo-piperazin können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 g |
| Schellack | 0.64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 g.

Beispiel 13: In analoger Weise wie in den Beispielen 11 und 12 beschrieben kann man auch pharmazeutische Präparate, enthaltend eine andere Verbindung der Formel I gemäss den Beispielen 1 bis 9 herstellen.

**Patentansprüche**

**Patentansprüche für folgende Vertragsstaaten : BE, CH, DE, FR, GB, IT, LI, LU, NL, SE**

**1.** Neue substituierte Pyrrolidin-2-one der Formel

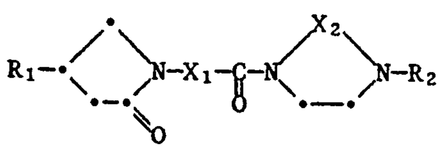

(I),

worin $R_1$ einen unsubstituierten oder durch Niederalkyl, Niederalkoxy, Halogen und/oder Trifluormethyl substituierten Phenyl- oder Naphthylrest bedeutet, $X_1$ für Niederalkyliden steht, $X_2$ Methylen, Aethylen oder Oxoäthylen bedeutet und $R_2$ Wasserstoff, Niederalkyl oder einen Rest der Formel

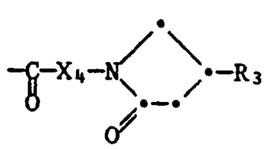

(Ia),

darstellt, in dem $X_4$ Niederalkyliden und $R_3$ Wasserstoff oder einen unsubstituierten oder durch Niederalkyl, Niederalkoxy, Halogen und/oder Trifluormethyl substituierten Phenyl- oder Naphthylrest bedeutet, in freier Form oder in Salzform, wobei unter mit "nieder" bezeichneten Resten solche zu verstehen sind, die bis und mit 7 C-Atome enthalten.

**2.** Verbindungen gemäss Anspruch 1 der Formel I, worin $R_1$ einen unsubstituierten oder durch $C_1$-$C_4$-Alkyl, $C_1$-$C_4$-Alkoxy, Halogen der Atomnummer bis und mit 35 und/oder Trifluormethyl mono- oder disubstituierten Phenyl- oder Naphthylrest bedeutet, $X_1$ $C_1$-$C_7$-Alkyliden darstellt, $X_2$ Methylen, Aethylen oder Oxoäthylen bedeutet und $R_2$ Wasserstoff, $C_1$-$C_7$-Alkyl oder eine Gruppe der Formel Ia bedeutet, in der $X_4$ $C_1$-$C_7$-Alkyliden und $R_3$ Wasserstoff oder einen unsubstituierten oder durch $C_1$-$C_4$-Alkyl, $C_1$-$C_4$-Alkoxy, Halogen der Atomnummer bis und mit 35 und/oder Trifluormethyl mono-oder disubstituierten Phenyl- oder Naphthylrest darstellt, und ihre Salze.

**3.** Verbindungen gemäss Anspruch 1 der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_7$-Alkyliden bedeutet, $X_2$ Oxoäthylen darstellt und $R_2$ Wasserstoff darstellt, und ihre Salze.

**4.** Verbindungen gemäss Anspruch 1 der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_4$-Alkyliden bedeutet, $X_2$ über die Carbonylgruppe mit der Teilstruktur -N($R_2$)- verbundenes Oxoäthylen darstellt und $R_2$ Wasserstoff darstellt, und ihre Salze.

**5.** Verbindungen gemäss Anspruch 1 der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_4$-Alkyliden bedeutet, $X_2$ Methylen oder Aethylen darstellt und $R_2$ $C_1$-$C_4$-Alkyl oder eine Gruppe der Formel Ia bedeutet, in der $X_4$ terminal gebundenes $C_1$-$C_4$-Alkyliden ist und $R_3$ Wasserstoff bedeutet, und ihre Salze.

**6.** Verbindungen gemäss Anspruch 1 der Formel I, worin $R_1$ unsubstituiertes oder durch Halogen der Atomnummer bis und mit 35, substituiertes Phenyl oder unsubstituiertes Naphthyl bedeutet, $X_1$ terminal gebundenes $C_1$-$C_4$-Alkyliden bedeutet, $X_2$ für über die Carbonylgruppe mit der Teilstruktur -N($R_2$)-verbundenes Oxoäthylen steht und $R_2$ Wasserstoff bedeutet, und ihre Salze.

**7.** Verbindungen gemäss Anspruch 1 der Formel I, worin $R_1$ unsubstituiertes oder durch Halogen der Atomnummer bis und mit 35, substituiertes Phenyl oder unsubstituiertes Naphthyl bedeutet, $X_1$ terminal gebundenes $C_1$-$C_7$-Alkyliden darstellt, $X_2$ über die Carbonylgruppe mit der Teilstruktur -N($R_2$)- verbundenes Oxoäthylen und $R_2$ Wasserstoff bedeutet, und ihre Salze.

**8.** 1-[4-(p-Chlorphenyl)-2-oxo-pyrrolidin-1-ylacetyl]-3-oxo-piperazin gemäss Anspruch 1 oder ein Salz davon.

**9.** 1-[4-(p-Fluorphenyl)-2-oxo-pyrrolidin-1-ylacetyl]-3-oxo-piperazin,
1-[4-(p-Chlorphenyl)-2-oxo-pyrrolidin-1-ylacetyl]-4-methyl-piperazin,
1-[2-(2-Oxo-4-phenyl-pyrrolidin-1-yl)acetyl]-piperazin,
1-[4-(p-Chlorphenyl)-2-oxo-pyrrolidin-1-ylacetyl]-4-(2-oxo-pyrrolidin-1-ylacetyl)-piperazin,
1-[4-(p-Chlorphenyl)-2-oxo-pyrrolidin-1-ylacetyl]-piperazin oder
1-[4-(p-Chlorphenyl)-2-oxo-pyrrolidin-1-ylacetyl]-4-methyl-3-oxo-piperazin gemäss Anspruch 1 oder jeweils ein Salz davon.

**10.** 1-{2-[4-(p-Chlorphenyl)-2-oxo-pyrrolidin-1-yl]-4-methyl-pentanoyl}-3-oxo-piperazin oder ein Salz davon.

**11.** Verbindungen gemäss einem der Ansprüche 1 und 4-6, 8 und 9 zur Anwendung in einem Verfahren zur Behandlung des menschlichen oder tierischen Körpers.

**12.** Verbindungen gemäss einem der Ansprüche 2, 3, 7 und 10 zur Anwendung in einem Verfahren zur Behandlung des menschlichen oder tierischen Körpers.

**13.** Pharmazeutische Präparate, enthaltend eine Verbindung gemäss einem der Ansprüche 1, 4-6, 8, 9 und 11 neben üblichen pharmazeutischen Hilfsstoffen.

**14.** Pharmazeutische Präparate, enthaltend eine Verbindung gemäss einem der Ansprüche 2, 3, 7, 10 und 12.

**15.** Verfahren zur Herstellung substituierter Pyrrolidin-2-one gemäss Anspruch 1 in freier Form oder in Salzform, dadurch gekennzeichnet, dass man
a) Verbindungen der Formeln

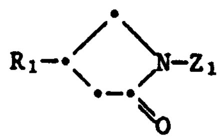 (II) und 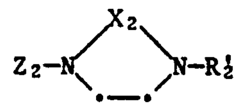 (III)

worin $Z_1$ eine Gruppe der Formel $-X_1-C(=O)-Z_3$ (IIa) und $Z_2$ Wasserstoff oder $Z_1$ Wasserstoff und $Z_2$ eine Gruppe der Formel $-C(=O)-X_1-Z_4$ (IIIa) darstellt, wobei $Z_3$ und $Z_4$ jeweils abspaltbare Reste bedeuten und $R_2'$ einen Rest $R_2$ oder eine Aminoschutzgruppe darstellt, oder deren Salze miteinander kondensiert oder
b) eine Verbindung der Formel

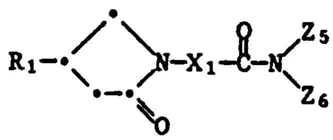 (IV),

worin $Z_5$ eine Grupee der Formeln $-CH_2-CH_2-N(R_2')-CH_2-Z_4$ (IVa),
$-CH_2-CH_2-N(R_2')-CH_2CH_2-Z_4$ (IVb),
$-CH_2-CH_2-N(R_2')-C(=O)-CH_2-Z_4$ (IVc) oder
$-CH_2-CH_2-N(R_2')-CH_2-C(=O)-Z_3$ (IVd) und $Z_6$ Wasserstoff bedeutet oder $Z_5$ eine Gruppe der Formel $-X_2-N(R_2')-H$ (IVe) und $Z_6$ eine Gruppe der Formel $-CH_2CH_2-Z_4$ (IVg) bzw. $Z_5$ eine Gruppe der Formel $-CH_2CH_2-N(R_2')-H$ (IVh) und $Z_6$ eine Gruppe der Formel $-CH_2-Z_4$ (IVi), $-CH_2CH_2-Z_4$ - (IVg), $-C(=O)-CH_2-Z_4$ (IVj) oder $-CH_2-C(=O)-Z_3$ (IVk) darstellt oder $Z_5$ für eine Gruppe der Formel $-CH_2-CN$ (IVl) und $Z_6$ für eine Gruppe der Formel $-CH_2CH_2-OH$ (IVm) bzw. $-C(=O)-CH_2-OH$ (IVn) steht, oder deren Salze oder eine Verbindung der Formel

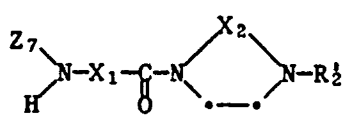 (V)

worin $Z_7$ eine Gruppe der Formeln

$-CH_2-CH(R_1)-CH_2-C(=O)-Z_3$ (Va) oder
$-C(=O)-CH_2-CH(R_1)-CH_2-Z_4$ (Vb)

bedeutet, oder deren Salze intramolekular cyclisiert, wobei $Z_3$ bzw. $Z_4$ jeweils abspeltbare Reste bedeuten und $R_2'$ einen Rest $R_2$ oder eine Aminoschutzgruppe darstellt, oder
c) Verbindungen der Formeln $R_1-CH(CH_2Z_4)-CH_2-C(=O)-Z_3$ (VIa) bzw.

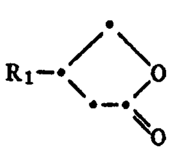 (VIb) und 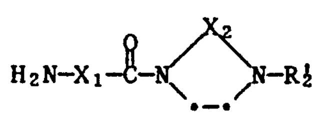 (VII),

miteinander kondensiert und jeweils eine gegebenenfalls vorhandene Aminoschutzgruppe $R_2'$ abspaltet, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I überführt, ein verfahrensgemäss erhältliches Enantiomeren- oder Diastereomerengemisch in die Komponenten aufspaltet und/oder eine verfahrensgemäss erhaltliche freie Verbindung in ein Salz oder ein verfahrensgemäss erhältliches Salz in die freie Verbindung oder in ein anderes Salz überführt.

**16.** Verwendung von Verbindungen gemäss einem der Ansprüche 1-10 als nootropes Arzneimittel oder zur Herstellung eines solchen.

**Patentansprüche für folgende Vertragsstaaten : AT, ES, GR**

**1.** Verfahren zur Herstellung substituierter Pyrrolidin-2-one der Formel

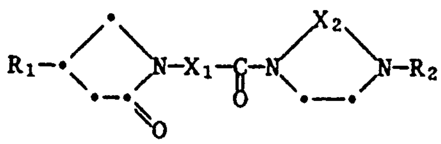

(I),

worin $R_1$ einen unsubstituierten oder durch Niederalkyl, Niederalkoxy, Halogen und/oder Trifluormethyl substituierten Phenyl- oder Naphthylrest bedeutet, $X_1$ für Niederalkyliden steht, $X_2$ Methylen, Aethylen oder Oxoäthylen bedeutet und $R_2$ Wasserstoff, Niederalkyl oder einen Rest der Formel

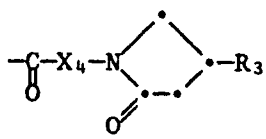

(Ia),

darstellt, in dem $X_4$ Niederalkyliden und $R_3$ Wasserstoff oder einen unsubstituierten oder durch Niederalkyl, Niederalkoxy, Halogen und/oder Trifluormethyl substituierten Phenyl- oder Naphthylrest bedeutet, in freier Form oder in Salzform, wobei unter mit "neider" bezeichneten Resten solche zu verstehen sind, die bis und mit 7 C-Atome enthalten, dadurch gekennzeichnet, dass man

a) Verbindungen der Formeln

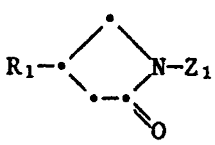

(II) und

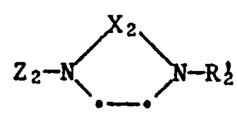

(III)

worin $Z_1$ eine Gruppe der Formel $-X_1-C(=O)-Z_3$ (IIa) und $Z_2$ Wasserstoff oder $Z_1$ Wasserstoff und $Z_2$ eine Gruppe der Formel $-C(=O)-X_1-Z_4$ (IIIa) darstellt, wobei $Z_3$ und $Z_4$ jeweils abspaltbare Reste bedeuten und $R_2'$ einen Rest $R_2$ oder eine Aminoschutsgruppe darstellt, oder deren Salze miteinander kondensiert oder

b) eine Verbindung der Formel

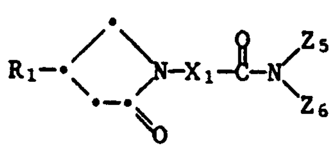

(IV),

worin $Z_5$ eine Grupee der Formeln $-CH_2-CH_2-N(R_2')-CH_2-Z_4$ (IVa),
$-CH_2-CH_2-N(R_2')-CH_2CH_2-Z_4$ (IVb),
$-CH_2-CH_2-N(R_2')-C(=O)-CH_2-Z_4$ (IVc) oder
$-CH_2-CH_2-N(R_2')-CH_2-C(=O)-Z_3$ (IVd) und $Z_6$ Wasserstoff bedeutet oder $Z_5$ eine Gruppe der Formel $-X_2-N(R_2')-H$ (IVe) und $Z_6$ eine Gruppe der Formel $-CH_2CH_2-Z_4$ (IVg) bzw. $Z_5$ eine Gruppe der Formel $-CH_2CH_2-N(R_2')-H$ (IVh) und $Z_6$ eine Gruppe der Formel $-CH_2-Z_4$ (IVi), $-CH_2CH_2-Z_4$ - (IVg), $-C(=O)-CH_2-Z_4$ (IVj) oder $-CH_2-C(=O)-Z_3$ (IVk) darstellt oder $Z_5$ für eine Gruppe der Formel

$-CH_2-CN$ (IVl) und $Z_6$ für eine Gruppe der Formel $-CH_2CH_2-OH$ (IVm) bzw. $-C(=O)-CH_2-OH$ (IVn) steht, oder deren Salze oder eine Verbindung der Formel

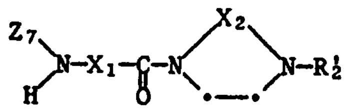
(V)

worin $Z_7$ eine Gruppe der Formeln

$-CH_2-CH(R_1)-CH_2-C(=O)-Z_3$ (Va) oder
$-C(=O)-CH_2-CH(R_1)-CH_2-Z_4$ (Vb)

bedeutet, oder deren Salze intramolekular cyclisiert, wobei $Z_3$ bzw. $Z_4$ jeweils abspaltbare Reste bedeuten und $R_2'$ einen Rest $R_2$ oder eine Aminoschutzgruppe darstellt, oder
c) Verbindungen der Formeln $R_1-CH(CH_2Z_4)-CH_2-C(=O)-Z_3$ (VIa) bzw.

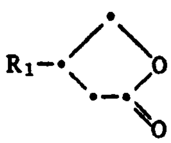
(VIb) und

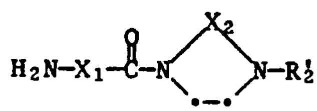
(VII),

miteinander kondensiert und jeweils eine gegebenenfalls vorhandene Aminoschutzgruppe $R_2'$ abspaltet, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I überführt, ein verfahrensgemäss erhältliches Enantiomeren- oder Diastereomerengemisch in die Komponenten aufspaltet und/oder eine verfahrensgemäss erhältliche freie Verbindung in ein Salz oder ein verfahrensgemäss erhältliches Salz in die freie Verbindung oder in ein anderes Salz überführt.

**2.** Verfahren gemäss Anspruch 1 zur Herstellung von Verbindungen der Formel I, worin $R_1$ einen unsubstituierten oder durch $C_1$-$C_4$-Alkyl, $C_1$-$C_4$-Alkoxy, Halogen der Atomnummer bis und mit 35 und/oder Trifluormethyl mono- oder disubstituierten Phenyl- oder Naphthylrest bedeutet, $X_1$ $C_1$-$C_7$-Alkyliden darstellt, $X_2$ Methylen, Aethylen oder Oxoäthylen bedeutet und $R_2$ Wasserstoff, $C_1$-$C_7$-Alkyl oder eine Gruppe der Formel Ia bedeutet, in der $X_4$ $C_1$-$C_7$-Alkyliden und $R_3$ Wasserstoff oder einen unsubstituierten oder durch $C_1$-$C_4$-Alkyl, $C_1$-$C_4$-Alkoxy, Halogen der Atomnummer bis und mit 35 und/oder Trifluormethyl mono-oder disubstituierten Phenyl- oder Naphthylrest darstellt, und ihrer Salze.

**3.** Verfahren gemäss Anspruch 1 zur Herstellung von Verbindungen der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_7$-Alkyliden bedeutet, $X_2$ Oxoäthylen darstellt und $R_2$ Wasserstoff darstellt, und ihrer Salze.

**4.** Verfahren gemäss Anspruch 1 zur Herstellung von Verbindungen der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_4$-Alkyliden bedeutet, $X_2$ über die Carbonylgruppe mit der Teilstruktur $-N(R_2)-$ verbundenes Oxoäthylen darstellt und $R_2$ Wasserstoff darstellt, und ihrer Salze.

**5.** Verfahren gemäss Anspruch 1 zur Herstellung von Verbindungen der Formel I, worin $R_1$ unsubstituiertes oder durch Halogen der Atomnummer bis und mit 35, $C_1$-$C_4$-Alkoxy, $C_1$-$C_4$-Alkyl oder Trifluormethyl substituiertes Phenyl oder unsubstituiertes Naphthyl bedeutet, $X_1$ terminal gebundenes $C_1$-$C_7$-Alkyliden bedeutet, $X_2$ Methylen oder Aethylen darstellt und $R_2$ $C_1$-$C_4$-Alkyl oder eine Gruppe der Formel Ia bedeutet, in der $X_4$ terminal gebundenes $C_1$-$C_4$-Alkyliden ist und $R_3$ Wasserstoff bedeutet,

und ihrer Salze.

6. Verfahren gemäss Anspruch 1 zur Herstellung von Verbindungen der Formel I, worin $R_1$ unsubstituiertes oder durch Halogen der Atomnummer bis und mit 35, substituiertes Phenyl oder unsubstituiertes Naphthyl bedeutet, $X_1$ terminal gebundenes $C_1$-$C_4$-Alkyliden bedeutet, $X_2$ für über die Carbonylgruppe mit der Teilstruktur -N($R_2$)- verbundenes Oxoäthylen steht und $R_2$ Wasserstoff bedeutet, und ihrer Salze.

7. Verfahren gemäss Anspruch 1 zur Herstellung von Verbindungen der Formel I, worin $R_1$ unsubstituiertes oder durch Halogen der Atomnummer bis und mit 35, substituiertes Phenyl oder unsubstituiertes Naphthyl bedeutet, $X_1$ terminal gebundenes $C_1$-$C_7$-Alkyliden darstellt, $X_2$ über die Carbonylgruppe mit der Teilstruktur -N($R_2$)- verbundenes Oxoäthylen und $R_2$ Wasserstoff bedeutet, und ihrer Salze.

8. Verfahren gemäss Anspruch 1 zur Herstellung von l-[4-(p-Chlorphenyl)-2-oxo-pyrrolidin-1-ylacetyl]-3-oxo-piperazin oder eines Salzes davon.

9. Verfahren gemäss Anspruch 1 zur Herstellung von 1-[4-(p-Fluorphenyl)-2-oxo-pyrrolidin-1-ylacetyl]-3-oxo-piperazin,
1-[4-(p-Chlorphenyl)-2-oxo-pyrrolidin-1-ylacetyl]-4-methyl-piperazin,
1-[2-(2-Oxo-4-phenyl-pyrrolidin-1-yl)acetyl]-piperizin,
1-[4-(p-Chlorphenyl)-2-oxo-pyrrolidin-1-ylacetyl]-4-(2-oxo-pyrrolidin-1-ylacetyl)-piperazin,
1-[4-(p-Chlorphenyl)-2-oxo-pyrrolidin-1-ylacetyl]-piperazin,
1-{2-[4-(p-Chlorphenyl)-2-oxo-pyrrolidin-1-yl]-4-methyl-pentanoyl}-3-oxo-piperazin oder
1-[4-(p-Chlorphenyl)-2-oxo-pyrrolidin-1-ylacetyl]-4-methyl-3-oxo-piperazin oder jeweils eines Salzes davon.

10. Verfahren zur Herstellung pharmazeutischer Präparate, dadurch gekenanzeichnet, dass man eine Verbindung, erhältlich gemäss einem der Ansprüche 1 bis 9, mit üblichen pharmazeutischen Hilfsstoffen vermischt.

## Claims

## Claims for the following Contracting States : BE, CH, DE, FR, GB, IT, LI, LU, NL, SE

1. A novel substituted pyrrolidin-2-one of the formula

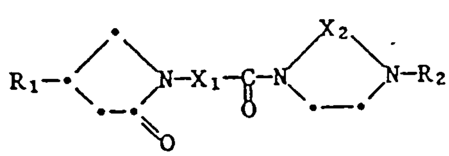

(I)

wherein $R_1$ represents a phenyl or naphthyl radical that is unsubstituted or substituted by lower alkyl, lower alkoxy, halogen and/or by trifluoromethyl, $X_1$ represents lower alkylidene, $X_2$ represents methylene, ethylene or oxoethylene and $R_2$ represents hydrogen, lower alkyl or a radical of the formula

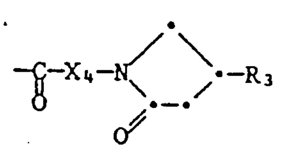

(Ia)

wherein $X_4$ represents lower alkylidene and $R_3$ represents hydrogen or a phenyl or naphthyl radical that is unsubstituted or substituted by lower alkyl, lower alkoxy, halogen and/or by trifluoromethyl, in free form or in the form of a salt, radicals termed "lower" being those that contain up to and including 7 carbon atoms.

2. A compound according to claim 1 of the formula I, wherein $R_1$ represents a phenyl or naphthyl radical that is unsubstituted or mono- or di-substituted by $C_1$-$C_4$-alkyl, $C_1$-$C_4$alkoxy, halogen having an atomic

number of up to and including 35 and/or by trifluoromethyl, $X_1$ represents $C_1$-$C_7$alkylidene, $X_2$ represents methylene, ethylene or oxoethylene and $R_2$ represents hydrogen, $C_1$-$C_7$alkyl or a group of the formula Ia wherein $X_4$ represents $C_1$-$C_7$alkylidene and $R_3$ represents hydrogen or a phenyl or naphthyl radical that is unsubstituted or mono- or di-substituted by $C_1$-$C_4$alkyl, $C_1$-$C_4$alkoxy, halogen having an atomic number of up to and including 35 and/or by trifluoromethyl, or a salt thereof.

**3.** A compound according to claim 1 of the formula I, wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_7$alkylidene, $X_2$ represents oxoethylene and $R_2$ represents hydrogen, or a salt thereof.

**4.** A compound according to claim 1 of the formula I, wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_4$alkylidene, $X_2$ represents oxoethylene bonded via the carbonyl group to the partial structure -N($R_2$)- and $R_2$ represents hydrogen, or a salt thereof.

**5.** A compound according to claim 1 of the formula I, wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_4$alkylidene, $X_2$ represents methylene or ethylene and $R_2$ represents $C_1$-$C_4$alkyl or a group of the formula Ia wherein $X_4$ is terminally bonded $C_1$-$C_4$alkylidene and $R_3$ represents hydrogen, or a salt thereof.

**6.** A compound according to claim 1 of the formula I, wherein $R_1$ represents phenyl that is unsubstituted or substituted by halogen having an atomic number of up to and including 35 or represents unsubstituted naphthyl, $X_1$ represents terminally bonded $C_1$-$C_4$alkylidene, $X_2$ represents oxoethylene bonded via the carbonyl group to the partial structure -N($R_2$)- and $R_2$ represents hydrogen, or a salt thereof.

**7.** A compound according to claim 1 of the formula I, wherein $R_1$ represents phenyl that is unsubstituted or substituted by halogen having an atomic number of up to and including 35 or represents unsubstituted naphthyl, $X_1$ represents terminally bonded $C_1$-$C_7$alkylidene, $X_2$ represents oxoethylene bonded via the carbonyl group to the partial structure -N($R_2$)- and $R_2$ represents hydrogen, or a salt thereof.

**8.** 1-[4-(p-chlorophenyl)-2-oxopyrrolidin-1-ylacetyl]-3-oxopiperazine according to claim 1 or a salt thereof.

**9.** 1-[4-(p-fluorophenyl)-2-oxopyrrolidin-1-ylacetyl]-3-oxopiperazine, 1-[4-(p-chlorophenyl)-2-oxopyrrolidin-1-ylacetyl]-4-methylpiperazine, 1-[2-(2-oxo-4-phenylpyrrolidin-1-yl)acetyl]-piperazine, 1-[4-(p-chlorophenyl)-2-oxopyrrolidin-1-ylacetyl]-4-(2-oxopyrrolidin-1-ylacetyl)-piperazine, 1-[4-(p-chlorophenyl)-2-oxopyrrolidin-1-ylacetyl]-piperazine or 1-[4-(p-chlorophenyl)-2-oxopyrrolidin-1-ylacetyl]-4-methyl-3-oxopiperazine according to claim 1 or a salt thereof in each case.

**10.** 1-{2-[4-(p-chlorophenyl)-2-oxopyrrolidin-1-yl]-4-methylpentanoyl}-3-oxopiperazine or a salt thereof.

**11.** A compound according to any one of claims 1 and 4 to 6, 8 and 9 for use in a method for the treatment of the human or animal body.

**12.** A compound according to any one of claims 2, 3, 7 and 10 for use in a method for the treatment of the human or animal body.

**13.** A pharmaceutical composition comprising a compound according to any one of claims 1, 4 to 6, 8, 9 and 11 together with customary pharmaceutical excipients.

**14.** A pharmaceutical composition comprising a compound according to any one of claims 2, 3, 7, 10 and 12.

**15.** A process for the preparation of a substituted pyrrolidin-2-one according to claim 1 in free form or in the form of a salt, wherein

a) compounds of the formulae

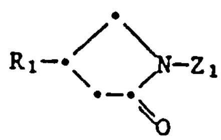 (II) and 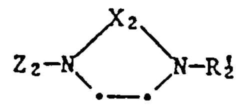 (III)

wherein $Z_1$ represents a group of the formula $-X_1-C(=O)-Z_3$ (IIa) and $Z_2$ represents hydrogen, or $Z_1$ represents hydrogen and $Z_2$ represents a group of the formula $-C(=O)-X_1-Z_4$ (IIIa), wherein each of $Z_3$ and $Z_4$ represents a removable radical, and $R_2'$ represents a radical $R_2$ or an amino-protecting group, or the salts thereof, are condensed with one another, or

b) a compound of the formula

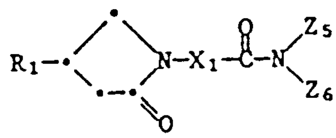 (IV)

wherein $Z_5$ represents a group of the formula $-CH_2-CH_2-N(R_2')-CH_2-Z_4$ (IVa), $-CH_2-CH_2-N(R_2')-CH_2CH_2-Z_4$ (IVb), $-CH_2-CH_2-N(R_2')-C(=O)-CH_2-Z_4$ (IVc) or $-CH_2-CH_2-N(R_2')-CH_2-C(=O)-Z_3$ (IVd) and $Z_6$ represents hydrogen, or $Z_5$ represents a group of the formula $-X_2-N(R_2')-H$ (IVe) and $Z_6$ represents a group of the formula $-CH_2CH_2-Z_4$ (IVg), or $Z_5$ represents a group of the formula $-CH_2CH_2-N(R_2')-H$ (IVh) and $Z_6$ represents a group of the formula $-CH_2-Z_4$ (IVi), $-CH_2CH_2-Z_4$ (IVg), $-C(=O)-CH_2-Z_4$ (IVj) or $-CH_2-C(=O)-Z_3$ (IVk), or $Z_5$ represents a group of the formula $-CH_2-CN$ (IVl) and $Z_6$ represents a group of the formula $-CH_2CH_2-OH$ (IVm) or $-C(=O)-CH_2-OH$ (IVn), or a salt thereof, or a compound of the formula

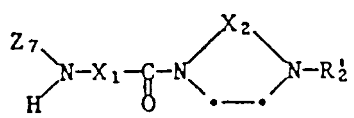 (V)

wherein $Z_7$ represents a group of the formula $-CH_2-CH(R_1)-CH_2-C(=O)-Z_3$ (Va) or $-C(=O)-CH_2-CH(R_1)-CH_2-Z_4$ (Vb), or a salt thereof, is cyclised intramolecularly, each of $Z_3$ and $Z_4$ representing a removable radical and $R_2'$ representing a radical $R_2$ or an amino-protecting group, or

c) compounds of the formulae $R_1-CH(CH_2Z_4)-CH_2-C(=O)-Z_3$ (VIa), or

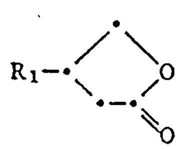 (VIb) and 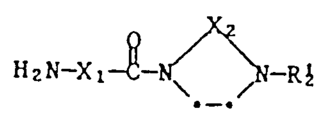 (VII)

are condensed with one another and, in each case, an amino-protecting group $R_2'$ which may be present is removed, if necessary an isomeric mixture obtainable in accordance with the process is separated into its components and the isomer of the formula I is isolated and, if desired, a compound obtainable in accordance with the process is converted into a different compound of the formula I, an enantiomeric or diastereoisomeric mixture obtainable in accordance with the process is separated into its components, and/or a free compound obtainable in accordance with the process is converted into a salt, or a salt obtainable in accordance with the process is converted into the free compound or into a different salt.

**16.** The use of a compound according to any one of claims 1 to 10 as a nootropic medicament or for the preparation of such a medicament.

**Claims for the following Contracting States : AT, ES, GR**

**1.** A process for the preparation of a substituted pyrrolidin-2-one of the formula

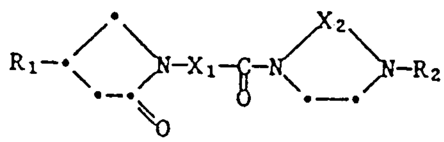

(I)

wherein $R_1$ represents a phenyl or naphthyl radical that is unsubstituted or substituted by lower alkyl, lower alkoxy, halogen and/or by trifluoromethyl, $X_1$ represents lower alkylidene, $X_2$ represents methylene, ethylene or oxoethylene and $R_2$ represents hydrogen, lower alkyl or a radical of the formula

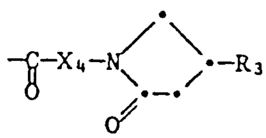

(Ia)

wherein $X_4$ represents lower alkylidene and $R_3$ represents hydrogen or a phenyl or naphthyl radical that is unsubstituted or substituted by lower alkyl, lower alkoxy, halogen and/or by trifluoromethyl, in free form or in the form of a salt, radicals termed "lower" being those that contain up to and including 7 carbon atoms, wherein

a) compounds of the formulae

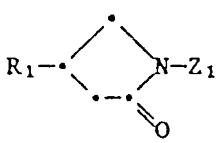

(II) and

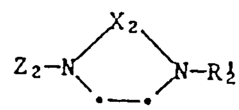

(III)

wherein $Z_1$ represents a group of the formula $-X_1-C(=O)-Z_3$ (IIa) and $Z_2$ represents hydrogen, or $Z_1$ represents hydrogen and $Z_2$ represents a group of the formula $-C(=O)-X_1-Z_4$ (IIIa), wherein each of $Z_3$ and $Z_4$ represents a removable radical, and $R_2'$ represents a radical $R_2$ or an amino-protecting group, or the salts thereof, are condensed with one another, or

b) a compound of the formula

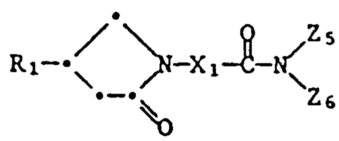

(IV)

wherein $Z_5$ represents a group of the formula $-CH_2-CH_2-N(R_2')-CH_2-Z_4$ (IVa), $-CH_2-CH_2-N(R_2')-CH_2CH_2-Z_4$ (IVb), $-CH_2-CH_2-N(R_2')-C(=O)-CH_2-Z_4$ (IVc) or $-CH_2-CH_2-N(R_2')-CH_2-C(=O)-Z_3$ (IVd) and $Z_6$ represents hydrogen, or $Z_5$ represents a group of the formula $-X_2-N(R_2')-H$ (IVe) and $Z_6$ represents a group of the formula $-CH_2CH_2-Z_4$ (IVg), or $Z_5$ represents a group of the formula $-CH_2CH_2-N(R_2')-H$ (IVh) and $Z_6$ represents a group of the formula $-CH_2-Z_4$ (IVi), $-CH_2CH_2-Z_4$ (IVg), $-C(=O)-CH_2-Z_4$ (IVj) or $-CH_2-C(=O)-Z_3$ (IVk), or $Z_5$ represents a group of the formula $-CH_2-CN$ (IVl) and $Z_6$ represents a group of the formula $-CH_2CH_2-OH$ (IVm) or $-C(=O)-CH_2-OH$ (IVn), or a salt thereof, or a compound of the formula

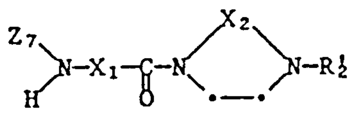 (V)

wherein $Z_7$ represents a group of the formula $-CH_2-CH(R_1)-CH_2-C(=O)-Z_3$ (Va) or $-C(=O)-CH_2-CH(R_1)-CH_2-Z_4$ (Vb), or a salt thereof, is cyclised intramolecularly, each of $Z_3$ and $Z_4$ representing a removable radical and $R_2'$ representing a radical $R_2$ or an amino-protecting group, or
c) compounds of the formulae $R_1-CH(CH_2Z_4)-CH_2-C(=O)-Z_3$ (VIa), or

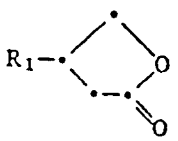 (VIb) and 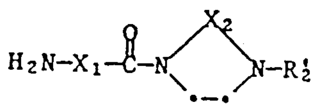 (VII)

are condensed with one another and, in each case, an amino-protecting group $R_2'$ which may be present is removed, if necessary an isomeric mixture obtainable in accordance with the process is separated into its components and the isomer of the formula I is isolated and, if desired, a compound obtainable in accordance with the process is converted into a different compound of the formula I, an enantiomeric or diastereoisomeric mixture obtainable in accordance with the process is separated into its components, and/or a free compound obtainable in accordance with the process is converted into a salt, or a salt obtainable in accordance with the process is converted into the free compound or into a different salt.

**2.** A process according to claim 1 for the preparation of a compound of the formula I wherein $R_1$ represents a phenyl or naphthyl radical that is unsubstituted or monoor di-substituted by $C_1$-$C_4$alkyl, $C_1$-$C_4$alkoxy, halogen having an atomic number of up to and including 35 and/or by trifluoromethyl, $X_1$ represents $C_1$-$C_7$alkylidene, $X_2$ represents methylene, ethylene or oxoethylene and $R_2$ represents hydrogen, $C_1$-$C_7$alkyl or a group of the formula Ia wherein $X_4$ represents $C_1$-$C_7$alkylidene and $R_3$ represents hydrogen or a phenyl or naphthyl radical that is unsubstituted or mono- or di-substituted by $C_1$-$C_4$alkyl,$C_1$-$C_4$alkoxy, halogen having an atomic number of up to and including 35 and/or by trifluoromethyl, or a salt thereof.

**3.** A process according to claim 1 for the preparation of a compound of the formula I wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_7$alkylidene, $X_2$ represents oxoethylene and $R_2$ represents hydrogen, or a salt thereof.

**4.** A process according to claim 1 for the preparation of a compound of the formula I wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_4$alkylidene, $X_2$ represents oxoethylene bonded via the carbonyl group to the partial structure $-N(R_2)-$ and $R_2$ represents hydrogen, or a salt thereof.

**5.** A process according to claim 1 for the preparation of a compound of the formula I wherein $R_1$ represents phenyl that is unsubstituted or substituted by halogen having an atomic number of up to and including 35, $C_1$-$C_4$alkoxy, $C_1$-$C_4$alkyl or by trifluoromethyl, or represents unsubstituted naphthyl, $X_1$ represents terminally bonded $C_1$-$C_7$alkylidene, $X_2$ represents methylene or ethylene and $R_2$ represents $C_1$-$C_4$alkyl or a group of the formula Ia wherein $X_4$ is terminally bonded $C_1$-$C_4$alkylidene and $R_3$ represents hydrogen, or a salt thereof.

**6.** A process according to claim 1 for the preparation of a compound of the formula I wherein $R_1$ represents phenyl that is unsubstituted or substituted by halogen having an atomic number of up to and

including 35 or represents unsubstituted naphthyl, $X_1$ represents terminally bonded $C_1$-$C_4$ alkylidene, $X_2$ represents oxoethylene bonded via the carbonyl group to the partial structure -N($R_2$)- and $R_2$ represents hydrogen, or a salt thereof.

**7.** A process according to claim 1 for the preparation of a compound of the formula I wherein $R_1$ represents phenyl that is unsubstituted or substituted by halogen having an atomic number of up to and including 35 or represents unsubstituted naphthyl, $X_1$ represents terminally bonded $C_1$-$C_7$ alkylidene, $X_2$ represents oxoethylene bonded via the carbonyl group to the partial structure -N($R_2$)- and $R_2$ represents hydrogen, or a salt thereof.

**8.** A process according to claim 1 for the preparation of 1-[4-(p-chlorophenyl)-2-oxopyrrolidin-1-ylacetyl]-3-oxopiperazine or a salt thereof.

**9.** A process according to claim 1 for the preparation of 1-[4-(p-fluorophenyl)-2-oxopyrrolidin-1-ylacetyl]-3-oxopiperazine, 1-[4-(p-chlorophenyl)-2-oxopyrrolidin-1-ylacetyl]-4-methylpiperazine, 1-[2-(2-oxo-4-phenylpyrrolidin-1-yl)acetyl]-piperazine, 1-[4-(p-chlorophenyl)-2-oxopyrrolidin-1-ylacetyl]-4-(2-oxopyrrolidin-1-ylacetyl)-piperazine, 1-[4-(p-chlorophenyl)-2-oxopyrrolidin-1-ylacetyl]-piperazine, 1-(2-[4-(p-chlorophenyl)-2-oxopyrrolidin-1-yl]-4-methylpentanoyl)-3-oxopiperazine or 1-[4-(p-chlorophenyl)-2-oxopyrrolidin-1-ylacetyl]-4-methyl-3-oxopiperazine or a salt thereof in each case.

**10.** A process for the preparation of a pharmaceutical composition, wherein a compound obtainable according to any one of claims 1 to 9 is mixed with customary pharmaceutical excipients.

**Revendications**

**Revendications pour les Etats contractants suivants : BE, CH, DE, FR, GB, IT, LI, LU, NL, SE**

**1.** Nouvelles pyrrolidine-2-ones substituées répondant à la formule

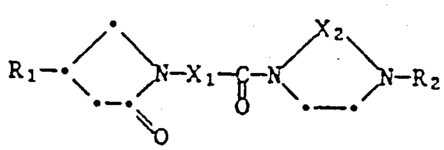

(I),

dans laquelle $R_1$ est un radical phényle ou naphtyle, non substitué ou substitué par des radicaux alkyle inférieur, alcoxy inférieur, halogéno et/ou trifluorométhyle, $X_1$ représente un radical alkylidène inférieur, $X_2$ est un radical méthylène, éthylène ou oxoéthylène, et $R_2$ est un atome d'hydrogène, un radical alkyle inférieur ou un radical de formule

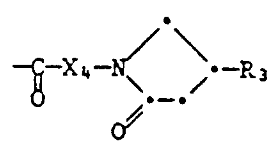

(Ia),

dans laquelle $X_4$ est un radical alkylidène inférieur et $R_3$ est un atome d'hydrogène ou un radical phényle ou naphtyle non substitué ou substitué par des radicaux alkyle inférieur, alcoxy inférieur, halogéno et/ou trifluorométhyle, sous forme libre ou sous forme d'un sel, le terme "inférieur" se rapportant à des radicaux ayant un nombre d'atomes de carbone inférieur ou égal à 7.

**2.** Composés selon la revendication 1, de formule I, dans lesquels $R_1$, est un radical phényle ou naphtyle, non substitué ou mono- ou di-substitué par des radicaux alkyle en $C_1$-$C_4$, alcoxy en $C_1$-$C_4$, halogéno ayant un numéro atomique inférieur ou égal à 35 et/ou trifluorométhyle, $X_1$ est un radical alkylidène en $C_1$-$C_7$, $X_2$ est un radical méthylène, éthylène ou oxoéthylène, et $R_2$ est un atome d'hydrogène, un radical alkyle en $C_1$-$C_7$ ou un groupe de formule Ia dans laquelle $X_4$ est un radical alkylidène en $C_1$-$C_7$ et $R_3$ est un atome d'hydrogène ou un radical phényle ou naphtyle, non substitué ou mono- ou di-substitué par dee radicaux alkyle en $C_1$-$C_4$, alcoxy en $C_1$-$C_4$, halogéno ayant un numéro atomique

inférieur ou égal à 35, et/ou trifluorométhyle, et leurs sels.

**3.** Composés selon la revendication 1, de formule I, dans lesquels $R_1$ est un radical phényle non substitué ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_7$ à liaison terminale, $X_2$ est un radical oxoéthylène et $R_2$ est un atome d'hydrogène, et leurs sels.

**4.** Composés selon la revendication 1, de formule I, dans lesquels $R_1$ est un radical phényle non substitué, ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_4$ à liaison terminale, $X_2$ est un radical oxoéthylène lié à la structure partielle -N($R_2$)- par l'intermédiaire du groupe carbonyle, et $R_2$ est un atome d'hydrogène, et leurs sels.

**5.** Composés selon la revendication 1, de formule I, dans lesquels $R_1$ est un radical phényle non substitué ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_4$ à liaison terminale, $X_2$ est un radical méthylène ou éthylène, et $R_2$ est un radical alkyle en $C_1$-$C_4$ ou un groupe de formule Ia dans laquelle $X_4$ est un radical alkylidène en $C_1$-$C_4$ à liaison terminale et $R_3$ est un atome d'hydrogène, et leurs sels.

**6.** Composés selon la revendication 1, de formule I, dans lesquels $R_1$ est un radical phényle non substitué ou substitué par des radicaux halogéno ayant un numéro atomique inférieur ou égal à 35, ou encore naphtyle non substitué, $X_1$ est un radical alkylidène en $C_1$-$C_4$ à liaison terminale, $X_2$ est un radical oxoéthylène lié à la structure partielle -N($R_2$)- par l'intermédiaire du groupe carbonyle, et $R_2$ est un atome d'hydrogène, et leurs sels.

**7.** Composés selon la revendication 1, de formule I, dans lesquels $R_1$ est un radical phényle non substitué ou substitué par des radicaux halogéno ayant un numéro atomique inférieur ou égal à 35, ou encore naphtyle non substitué, $X_1$ est un radical alkylidène en $C_1$-$C_7$ à liaison terminale, $X_2$ est un radical oxoéthylène lié à la structure partielle -N($R_2$)- par l'intermédiaire du groupe carbonyle et $R_2$ est un atome d'hydrogène, et leurs sels.

**8.** 1-[4-(p-chlorophényl)-2-oxo-pyrrolidin-1-ylacétyl]-3-oxo-pipérazine selon la revendication 1, ou l'un de ses sels.

**9.** 1-[4-(p-fluorophényl)-2-oxo-pyrrolidin-1-ylacétyl]-3-oxo-pipérazine,
1-[4-(p-chlorophényl)-2-oxo-pyrrolidin-1-ylacétyl]-4-méthyl-pipérazine,
1-[2-(2-oxo-4-phényl-pyrrolidin-1-yl)acétyl]-pipérazine,
1-[4-(p-chlorophényl)-2-oxo-pyrrolidin-1-ylacétyl]-4-(2-oxo-pyrrolidin-1-ylacétyl)-pipérazine,
1-[4-(p-chlorophényl)-2-oxo-pyrrolidin-1-ylacétyl]-pipérazine ou
1-[4-(p-chlorophényl)-2-oxo-pyrrolidin-1-ylacétyl]-4-méthyl-3-oxo-pipérazine selon la revendication 1, ou un de leurs sels.

**10.** 1-{2-[4-(p-chlorophényl)-2-oxo-pyrrolidine-1-yl]-4-méthyl-pentanoyl}-3-oxo-pipérazine ou l'un de ses sels.

**11.** Composés selon l'une des revendications 1 et 4 à 6, 8 et 9, pour utilisation dans un procédé destiné au traitement de l'organisme humain ou animal.

**12.** Composés selon l'une des revendications 2, 3, 7 et 10, pour utilisation dans un procédé de traitement de l'organisme humain ou animal.

**13.** Préparations pharmaceutiques contenant un composé selon l'une des revendications 1, 4 à 6, 8, 9 et 11, en plus des substances auxiliaires pharmaceutiques usuelles.

**14.** Préparations pharmaceutiques contenant un composé selon l'une des revendications 2, 3, 7, 10 et 12.

**15.** Procédé de préparation de pyrrolidine-2-ones substituées selon la revendication 1, sous forme libre ou sous forme d'un sel, caractérisé en ce que :

a) on condense l'un avec l'autre des composés de formules

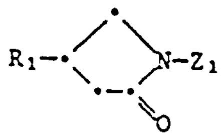 (II) et 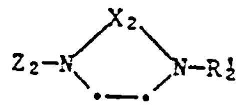 (III)

où $Z_1$ est un groupe de formule $-X_1-C(=O)-Z_3$ (IIa) et $Z_2$ est un atome d'hydrogène, ou encore $Z_1$ est un atome d'hydrogène et $Z_2$ est un groupe de formules $-C(=O)-X_1-Z_4$ (IIIa), $Z_3$ et $Z_4$ représentant chacun des radicaux éliminables, et $R_2$' étant un radical $R_2$' ou un groupe amino-protecteur, ou leurs sels, ou

b) on cyclise par cyclisation intra-moléculaire un composé de formule

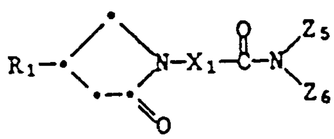 (IV),

dans laquelle $Z_5$ est un groupe de formule
$-CH_2-CH_2-N(R_2')-CH_2-Z_4$ (IVa),
$-CH_2-CH_2-N(R_2')-CH_2CH_2-Z_4$ (IVb)
$-CH_2-CH_2-N(R_2')-C(=O)-CH_2-Z_4$ (IVc) ou
$-CH_2-CH_2-N(R_2')-CH_2-C(=O)-Z_3$ (IVd)et $Z_6$ est un atome d'hydrogène, ou encore $Z_5$ est un groupe de formule $-X_2-N(R_2')-H$ (IVe) et $Z_6$ est un groupe de formule $-CH_2-CH_2-Z_4$ (IVg), ou encore $Z_5$ est un groupe de formule $-CH_2-CH_2-N(R_2')-H$ (IVh) et $Z_6$ est un groupe de formule $-CH_2-Z_4$ (IVi), $-CH_2-CH_2-Z_4$ (IVg), $-C(=O)-CH_2-Z_4$ (IVj) ou $-CH_2-C(=O)-Z_3$ (IVk), ou encore $Z_5$ représente un groupe de formule $-CH_2-CN$ (IVl) et $Z_6$ représente un groupe de formule $-CH_2CH_2-OH$ (IVm) ou $-C(=O)-CH_2-OH$ (IVn), ou ses sels, ou encore un composé de formule

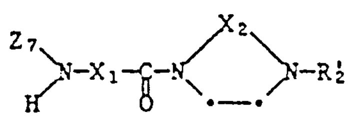 (V)

dans laquelle $Z_7$ est un groupe de formule

$-CH_2-CH(R_1)-CH_2-C(=O)-Z_3$ (Va) ou
$-C(=O)-CH_2-CH(R_1)-CH_2-Z_4$ (Vb),

ou ses sels, où $Z_3$ et $Z_4$ représentent chacun des radicaux éliminables, et $R_2$' est un radical $R_2$ ou représente un groupe amino-protecteur, ou bien

c) on condense l'un avec l'autre des composés de formules $R_1-CH(CH_2Z_4)-CH_2-C(=O)-Z_3$ (VIa) ou

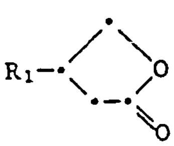 (VIb) et 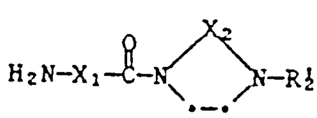 (VII),

et dans chaque cas on élimine un groupe amino-protecteur $R_2$' éventuellement présent, on sépare si

nécessaire en ses composants un mélange d'isomères pouvant être obtenu par le procédé selon l'invention et on isole l'isomère de formule I et, si on le désire, on convertit un composé pouvant être obtenu par un procédé selon l'invention en un autre composé de formule I, on dédouble en ses composants un mélange d'énantiomères ou de diastéréoisomères pouvant être obtenu par un procédé selon l'invention, et/ou on convertit en un sel un composé libre pouvant être obtenu par un procédé selon l'invention, ou en le composé libre ou en un autre sel, un sel pouvant être obtenu par un procédé selon l'invention.

**16.** Utilisation de composés selon l'une des revendications 1 à 10 en tant que médicament nootrope, ou pour la préparation d'un tel médicament.

**Revendications pour les Etats contractants suivants : AT, ES, GR**

**1.** Procédé de préparation de pyrrolidine-2-ones substituéees répondant à la formule

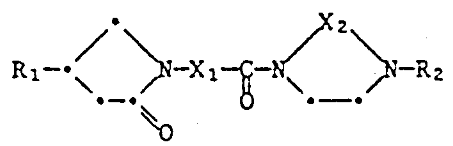

(I),

dans laquelle $R_1$ est un radical phényle ou naphtyle, non substitué ou substitué par des radicaux alkyle inférieur, alcoxy inférieur, halogéno et/ou trifluorométhyle, $X_1$ représente un radical alkylidène inférieur, $X_2$ est un radical méthylène, éthylène ou oxoéthylène, et $R_2$ est un atome d'hydrogène, un radical alkyle inférieur ou un radical de formule

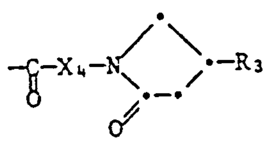

(Ia),

dans laquelle $X_4$ est un radical alkylidène inférieur et $R_3$ est un atome d'hydrogène ou un radical phényle ou naphtyle non substitué ou substitué par des radicaux alkyle inférieur, alcoxy inférieur, halogéno et/ou trifluorométhyle, sous forme libre ou sous forme d'un sel, le terme "inférieur" se rapportant à des radicaus ayant un nombre d'atomes de carbone inférieur ou égal à 7, caractérisé en ce que :

a) on condense l'un avec l'autre des composés de formules

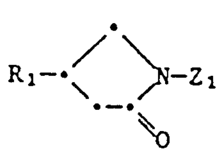

(II) et

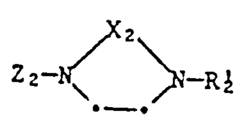

(III)

où $Z_1$ est un groupe de formule $-X_1-C(=O)-Z_3$ (IIa) et $Z_2$ est un atome d'hydrogène, ou encore $Z_1$ est un atome d'hydrogène et $Z_2$ est un groupe de formule $-C(=O)-X_1-Z_4$ (IIIa), $Z_3$ et $Z_4$ représentant chacun des radicaux éliminables, et $R_2'$ étant un radical $R_2$ ou un groupe amino-protecteur, ou leurs sels, ou

b) on cyclise par cyclisation intra-moléculaire un composé de formule

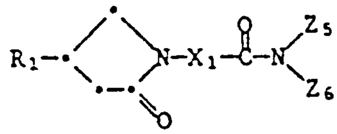

(IV),

dans laquelle $Z_5$ est un groupe de formule
$-CH_2-CH_2-N(R_2')-CH_2-Z_4$ (IVa),
$-CH_2-CH_2-N(R_2')-CH_2CH_2-Z_4$ (IVb),
$-CH_2-CH_2-N(R_2')-C(=O)-CH_2-Z_4$ (IVc) ou
$-CH_2-CH_2-N(R_2')-CH_2-C(=O)-Z_3$ (IVd)et $Z_6$ est un atome d'hydrogène, ou encore $Z_5$ est un groupe de formule $-X_2-N(R_2')-H$ (IVe) et $Z_6$ est un groupe de formule $-CH_2-CH_2-Z_4$ (IVg), ou encore $Z_5$ est un groupe de formule $-CH_2-CH_2-N(R_2')-H$ (IVh) et $Z_5$ est un groupe de formule $-CH_2-Z_4$ (IVi), $-CH_2-CH_2-Z_4$ (IVg), $-C(=O)-CH_2-Z_4$ (IVj) ou $-CH_2-C(=O)-Z_3$ (IVk), ou encore $Z_5$ représente un groupe de formule $-CH_2-CN$ (IVl) et $Z_6$ représente un groupe de formule $-CH_2CH_2-OH$ (IVm) ou $-C(=O)-CH_2-OH$ (IVn), ou ses sels, ou encore un composé de formule

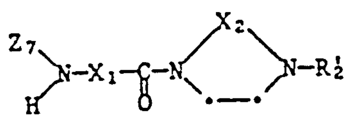

(V)

dans laquelle $Z_7$ est un groupe de formule

$-CH_2-CH(R_1)-CH_2-C(=O)-Z_3$ (Va) ou
$-C(=O)-CH_2-CH(R_1)-CH_2-Z_4$ (Vb),

ou ses sels, où $Z_3$ et $Z_4$ représentent chacun des radicaux éliminables, et $R_2'$ est un radical $R_2$ ou représente un groupe amino-protecteur, ou bien
c) on condense l'un avec l'autre des composés de formules $R_1-CH(CH_2Z_4)-CH_2-C(=O)-Z_3$ (VIa) ou

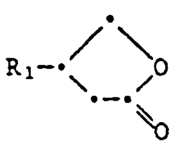

(VIb) et

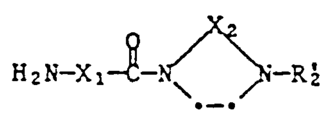

(VII),

et dans chaque cas on élimine un groupe amino-protecteur $R_{2'}$ éventuellement présent, on sépare si nécessaire en ses composants un mélange d'isomères pouvant être obtenu par le procédé selon l'invention et on isole l'isomère de formule I et, si on le désire, on convertit un composé pouvant être obtenu par un procédé selon l'invention en un autre composé de formule I, on dédouble en ses composants un mélange d'énantiomères ou de diastéréoisomères pouvant être obtenu par un procédé selon l'invention, et/ou on convertit en un sel un composé libre pouvant être obtenu par un procédé selon l'invention, ou en le composé libre ou en un autre sel, un sel pouvant être obtenu par un procédé selon l'invention.

**2.** Procédé selon la revendication 1, destiné à la préparation de composés de formule I, où $R_1$ est un radical phényle ou naphtyle, non substitué ou mono- ou di-substitué par des radicaux alkyle en $C_1$-$C_4$, alcoxy en $C_1$-$C_4$, halogéno ayant un numéro atomique inférieur ou égal à 35 et/ou trifluorométhyle, $X_1$ est un radical alkylidène en $C_1$-$C_7$, $X_2$ est un rédical méthylène, éthylène ou oxoéthylène, et $R_2$ est un atome d'hydrogène un radical alkyle en $C_1$-$C_7$ ou un groupe de formule Ia dans laquelle $X_4$ est un radical alkylidène en $C_1$-$C_7$ et $R_3$ est un atome d'hydrogène ou un radical phényle ou naphtyle, non substitué ou mono- ou di-substitué par des radicaux alkyle en $C_1$-$C_4$ alcoxy en $C_1$-$C_4$, halogéno ayant

un numéro atomique inférieur ou égal à 35, et/ou trifluorométhyle, et leurs sels.

**3.** Procédé selon la revendication 1, destiné à la préparation de composés de formule I, où $R_1$ est un radical phényle non substitué ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_7$ à liaison terminale, $X_2$ est un radical oxoéthylène et $R_2$ est un atome d'hydrogène, et leurs sels.

**4.** Procédé selon la revendication 1, destiné à la préparation de composés de formule I, où $R_1$ est un radical phényle non substitué ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_4$ à liaison terminale, $X_2$ est un radical oxoéthylène lié à la structure partielle -N($R_2$)- par l'intermédiaire du groupe carbonyle, et $R_2$ est un atome d'hydrogène, et leurs sels.

**5.** Procédé selon la revendication 1, destiné à la préparation de composés de formule I, où $R_1$ est un radical phényle non substitué ou substitué par des radicaux halogéno ayant un numéro atomique inférieur ou égal à 35, alcoxy en $C_1$-$C_4$, alkyle en $C_1$-$C_4$ ou trifluorométhyle, ou encore naphtyle non substitué, $X_1$ est un radical alkylidène en $C_1$-$C_4$ à liaison terminale, $X_2$ est un radical méthylène ou éthylène, et $R_2$ est un radical alkyle en $C_1$-$C_4$ ou un groupe de formule Ia dans laquelle $X_4$ est un radical alkylidène en $C_1$-$C_4$ à liaison terminale et $R_3$ est un atome d'hydrogène, et leurs sels.

**6.** Procédé selon la revendication 1, destiné à la préparation de composés de formule I, où $R_1$ est un radical phényle non substitué, ou substitué par des radicaux halogéno ayant un numéro atomique inférieur ou égal à 35, ou encore naphtyle non substitué, $X_1$ est un radical alkylidène en $C_1$-$C_4$ à liaison terminlae, $X_2$ est un radical oxoéthylène lié à la structure partielle -N($R_2$)- par l'intermédiaire du groupe carbonyle, et $R_2$ est un atome d'hydrogène, et leurs sels.

**7.** Procédé selon la revendication 1, destiné à la préparation de composés de formule I, où $R_1$ est un radical phényle non substitué ou substitué par des radicaux halogéno ayant un numéro atomique inférieur ou égal à 35, ou encore naphtyle non substitué, $X_1$ est un radical alkylidène en $C_1$-$C_7$ à liaison terminale, $X_2$ est un radical oxoéthylène lié à la structure partielle -N($R_2$)- par l'intermédiaire du groupe carbonyle et $R_2$ est un atome d'hydrogène, et leurs sels.

**8.** Procédé selon la revendication 1, destiné à la préparation de la 1-[4-(p-chlorophényl)-2-oxo-pyrrolidin-1-ylacétyl]-3-oxo-pipérazine ou d'un de ses sels.

**9.** Procédé selon la revendication 1, destiné à la préparation des composés suivants :
1-[4-(p-fluorophényl)-2-oxo-pyrrolidin-1-ylacétyl]-3-oxo-pipérazine,
1-[4-(p-chlorophényl)-2-oxo-pyrrolidin-1-ylacétyl]-4-méthyl-pipérazine,
1-[2-(2-oxo-4-phényl-pyrrolidin-1-yl)acétyl]pipérazine
1-[4-(p-chlorophényl)-2-oxo-pyrrolidin-1-ylacétyl]-4-(2-oxo-pyrrolidin-1-ylacétyl)-pipérazine,
1-[4-(p-chlorophényl)-2-oxo-pyrrolidin-1-ylacétyl]-pipérazine,
1-{2-[4-(p-chlorophényl)-2-oxo-pyrrolidin-1-yl]-4 méthyl-pentanoyl}-3-oxo-pipérazine, ou
1-[4-(p-chlorophényl)-2-oxo-pyrrolidin-1-ylacétyl]-4-méthyl-3-oxo-pipérazine, ou de l'un de leurs sels.

**10.** Procédé de fabrication de préparations pharmaceutiques, caractérisé en ce qu'on mélange à des produits auxiliaires pharmaceutiques usuels un composé pouvant être obtenu selon l'une des revendications 1 à 9.